# EUROPÄISCHE PATENTSCHRIFT

(11) **EP 2 535 071 B1**
(45) Veröffentlichungstag und Bekanntmachung des Hinweises auf die Patenterteilung: **13.06.2018**
(21) Anmeldenummer: 11170216.3
(22) Anmeldetag: 16.06.2011
(51) Int. Cl.: A61M 5/145, A61M 5/142

(54) **Medikationsvorrichtung zur dosierten Abgabe eines fluiden Mediums**
Medication device for metered discharge of a liquid media
Dispositif de médication pour la distribuer dosée d'un fluide

(43) Veröffentlichungstag der Anmeldung: 19.12.2012
(73) Patentinhaber: F. Hoffmann-La Roche AG, 4070 Basel (CH); Roche Diabetes Care GmbH, 68305 Mannheim (DE)
(72) Erfinder: Arnitz, Theo, 68753 Waghäusel (DE)
(74) Vertreter: Herzog, Fiesser & Partner Patentanwälte PartG mbB

(56) Entgegenhaltungen:
- EP-A1- 1 920 793
- WO-A1-03/103763
- WO-A2-2007/122621
- US-A- 4 155 362
- US-A1- 2005 159 708
- US-A1- 2010 204 659

## Beschreibung

### Gebiet der Erfindung

Die Erfindung betrifft eine Medikationsvorrichtung zur dosierten Abgabe eines fluiden Mediums nach Anspruch 1. Weiterhin betrifft die Erfindung ein Verfahren zur dosierten Abgabe eines fluiden Mediums nach Anspruch 18, sowie eine Verwendung der Medikationsvorrichtung nach Anspruch 19. Derartige Medikationsvorrichtungen, Verfahren und Verwendungen sind in vielen Bereichen der Naturwissenschaften, Technik und Medizintechnik einsetzbar. Ein besonderer Schwerpunkt, auf welchen die Erfindung jedoch nicht beschränkt ist, liegt in der dosierten Abgabe eines fluiden Therapeutikums und/oder Diagnostikums, beispielsweise in einer dosierten Abgabe von Insulin.

### Stand der Technik

Aus dem Stand der Technik ist eine Vielzahl von Medikationsvorrichtungen bekannt. Dabei wird eine Vielzahl von Antriebs- und Dosierkonzepten verwendet, welche grundsätzlich auch zum Teil im Rahmen der vorliegenden Erfindung zum Einsatz kommen können.

Ein erstes Konzept arbeitet grundsätzlich nach dem Prinzip eines Behälters zur Aufnahme des fluiden Mediums, in welchem ein Kolben gelagert ist. Dieser Kolben kann beispielsweise elektrisch angetrieben werden. Nach diesem Prinzip arbeiten viele am Markt befindliche Insulinpumpen. Ein anderes Konzept arbeitet mit federgelagerten Druckspeichern, welche von einem Benutzer beispielsweise manuell befüllt werden können.

Ein Beispiel für das erste der genannten Konzepte ist in WO 03/103763 A1 dargestellt. In dieser Schrift wird eine Vorrichtung zur Abgabe eines Fluids an einen Patienten offenbart. Mittels eines Kolbens wird das Fluid dosiert, wobei der Kolben durch einen integrierten Raupenantrieb vorgetrieben wird. Weiterhin wird beschrieben, dass mittels eines Barcodes und einer optischen Erfassungseinheit eine Abgabe einer Flüssigkeitsmenge überwacht werden kann.

Ein Beispiel des zweiten oben beschriebenen Konzepts ist in WO 2004/105827 A2 sowie in "The Spring™ Technology: Motor-Based Pump vs. Spring-Based Pump", erhältlich über www.springnow.com, beschrieben. Nach diesem Konzept wird ein durch eine Feder druckbeaufschlagtes Reservoir verwendet, welches über ein Einweg-Ventil befüllt werden kann. Weiterhin ist ein Abgabepfad für das Fluid vorgesehen, in welchem mittels einer Differenzdruckanordnung und mehreren Ventilen eine definierte Abgabemenge überwacht werden kann.

EP 1 920 793 A1 beschreibt eine Vorrichtung zur Bestimmung des Füllstandes einer Substanz in einer Ampulle mit mindestens einem an oder in der Nähe der Ampulle angeordneten lichtempfindlichen Sensor, mit welchem die Position eines Verdrängungskörpers in der Ampulle, welche sich an oder in der Nähe des Sensors befindet, detektiert werden kann sowie ein Verfahren zur Bestimmung des Füllstandes einer Substanz in einer Ampulle mit mindestens einem lichtempfindlichen Sensor, wobei anhand des auf den lichtempfindlichen Sensor auftreffenden und von einem in der Ampulle enthaltenen Verdrängungskörper bezüglich seiner optischen Eigenschaften oder Intensität veränderten Lichtes die Position des Verdrängungskörpers, der Vorderseite, und/oder der Rückseite des Verdrängungskörpers ermittelt wird.

Die aus dem Stand der Technik bekannten Konzepte weisen eine Mehrzahl technischer Herausforderungen auf. So wird üblicherweise bei angetriebenen Kolben ein Vortrieb des Kolbens vorgegeben. Hierdurch baut sich ein Druck in dem fluiden Medium auf, und ein Fluidtransfer erfolgt. Dabei können jedoch Hystereseeffekte auftreten, welche einen Einfluss auf die Dosiergenauigkeit haben können. Diese technische Herausforderung wird dadurch verschärft, dass, beispielsweise bei einer Dosierung von Medikamenten wie Insulin, eine hochgenaue Kontrolle der dosierten Menge in stark unterschiedlichen Bereichen erforderlich ist. So ist einerseits eine hochgenaue Dosierung bei niedrigen Dosierraten erforderlich, um beispielsweise eine Grundlast (Basalrate) zu kontrollieren. Im Wechsel hierzu müssen jedoch kurzfristig auch größere Dosiermengen hochpräzise abgegeben werden, um eine Spitzenlast (Bolus) gewährleisten zu können. Treten Hystereseeffekte oder auch Haftreibungseffekte auf, so kann dies zu erheblichen Problemen bei der Dosiergenauigkeit führen.

Eine weitere Herausforderung besteht darin, dass ein Vortrieb in der Regel über einen elektrischen Antrieb erfolgt, wodurch eine entsprechend dimensionierte Energieversorgung, beispielsweise über eine Batterie, erforderlich ist. Diese Energieversorgung bewirkt, dass bekannte Medikationsvorrichtungen in der Regel vergleichsweise großvolumig ausgestaltet werden müssen.

Eine weitere technische Herausforderung besteht darin, dass in der Regel vor einer Inbetriebsetzung und bei praktisch jeder Nachfüllung der Medikationsvorrichtung eine Entlüftung erforderlich ist. Werden beispielsweise bei üblichen Systemen Kartuschen ausgetauscht, so ist in der Regel eine Entlüftung (Priming) der gesamten Fluidik erforderlich. Insgesamt bewirkt dies, in Zusammenwirkung mit anderen technischen Herausforderungen, dass ein Anwender in der Regel viele Arbeitsschritte durchzuführen hat, was die Handhabbarkeit üblicher Systeme stark erschwert.

### Aufgabe der Erfindung

Es ist daher eine Aufgabe der Erfindung, eine Medikationsvorrichtung und ein Verfahren zur dosierten Abgabe eines fluiden Mediums bereitzustellen, welche die Nachteile bekannter Vorrichtungen und Verfahren der genannten Art zumindest weitgehend vermeiden. Insbesondere sollte die Handhabbarkeit des Systems verbessert werden, Volumen und Gewicht der Medikationsvorrichtung sollten reduziert werden, und die Dosiergenauigkeit sollte erhöht werden, insbesondere auch bei kleinen Dosiermengen.

### Offenbarung der Erfindung

Diese Aufgabe wird gelöst durch eine Medikationsvorrichtung, ein Verfahren und eine Verwendung mit den Merkmalen der unabhängigen Ansprüche. Vorteilhafte Weiterbildungen der Erfindung, welche einzeln oder in Kombination realisierbar sind, sind in den abhängigen Ansprüchen dargestellt.

Allgemein wird darauf hingewiesen, dass die Begriffe "aufweisen" und "umfassen" sowie entsprechende grammatikalische Abwandlungen dieser Verben im Rahmen der vorliegenden Erfindung sowohl abschließend als auch nicht-abschließend verstanden werden können. So kann der Ausdruck "A weist B auf oder der Ausdruck "A umfasst B" sowohl abschließend verstanden werden, dahingehend, dass A lediglich aus B besteht und neben B keine weiteren Elemente aufweist. Alternativ können die Ausdrücke auch nicht-abschließend verstanden werden, dahingehend, dass A neben B mindestens ein weiteres Element umfasst.

In einem ersten Aspekt der vorliegenden Erfindung wird eine Medikationsvorrichtung zur dosierten Abgabe eines fluiden Mediums vorgeschlagen. Unter einer Medikationsvorrichtung ist allgemein eine Vorrichtung zu verstehen, welche eingerichtet ist, um ein fluides Medium, also eine Flüssigkeit und/oder ein Gas, kontrolliert abzugeben, beispielsweise gesteuert durch ein entsprechendes Programm und/oder durch einen Benutzer gesteuert.

Unter einem fluiden Medium ist allgemein eine Flüssigkeit und/oder ein Gas zu verstehen. Auch eine Mischung mehrerer Flüssigkeiten und/oder Gase kann enthalten sein. Insbesondere kann das fluide Medium mindestens ein fluides Diagnostikum und/oder mindestens ein fluides Therapeutikum umfassen. Ohne Beschränkung weiterer möglicher Ausgestaltungen wird die Erfindung im Folgenden insbesondere unter Bezugnahme auf die Verwendung von Insulin als fluidem Medium beschrieben, sodass die Medikationsvorrichtung ohne Beschränkung weiterer möglicher Ausgestaltungen insbesondere als Insulinpumpe ausgestaltet sein kann. Grundsätzlich kann die Medikationsvorrichtung jedoch auf andere Weise ausgestaltet sein und kann, neben der Ausgestaltung zur dosierten Abgabe mindestens eines Therapeutikums und/oder Diagnostikums grundsätzlich auch zur Abgabe anderer Arten fluider Medien ausgestaltet sein, insbesondere für den Einsatz in den Naturwissenschaften und der Technik. Beispielsweise kann die Medikationsvorrichtung auch im Rahmen einer Prozesssteuerung chemischer Prozesse, beispielsweise bei einer Synthese und/oder einer Analyse, eingesetzt werden.

Unter einer dosierten Abgabe des fluiden Mediums wird im Rahmen der vorliegenden Erfindung allgemein eine Abgabe des fluiden Mediums verstanden, bei welcher eine abgegebene Menge und/oder eine Abgaberate (beispielsweise angegeben in Form eines Volumenstroms und/oder in Form eines Massenstroms) mindestens einer Vorgabe entspricht, wobei diese Vorgabe statisch oder auch variabel ausgestaltet werden kann.

Die Medikationsvorrichtung gemäß dem ersten Aspekt der vorliegenden Erfindung umfasst mindestens ein Transport- und Transferbehältnis. Unter einem Transport- und Transferbehältnis wird allgemein ein Behältnis verstanden, welches eingerichtet ist, um das fluide Medium zumindest vorübergehend aufzunehmen. In diesem Transport- und Transferbehältnis kann das fluide Medium beispielsweise gelagert werden und/oder vertrieben werden und/oder vorübergehend aufgenommen sein. Das Transport- und Transferbehältnis weist mindestens einen Behälter zur Aufnahme des fluiden Mediums auf. Unter einem Behälter ist allgemein eine Vorrichtung mit mindestens einem geschlossenen Innenraum zu verstehen, welcher vorzugsweise dicht gegenüber dem fluiden Medium ausgestaltet ist, sodass das fluide Medium in diesem Innenraum für einen Zeitraum von mindestens mehreren Minuten, vorzugsweise mindestens mehreren Stunden, insbesondere mehreren Tagen und besonders bevorzugt mindestens mehreren Wochen, Monaten oder sogar Jahren aufgenommen werden kann.

Der Behälter weist mindestens ein verschiebbares Element auf. Unter einem verschiebbaren Element wird dabei allgemein ein Element verstanden, welches relativ zu mindestens einem weiteren Bestandteil des Behälters in mindestens einer Dimension beweglich gelagert ist. Dieses verschiebbare Element kann beispielsweise relativ zu einer Behälterwand des Behälters in mindestens einer Dimension linear verschiebbar gelagert sein. Beispielsweise kann das verschiebbare Element mindestens einen Stopfen umfassen, welcher in einem Innenraum des Behälters verschiebbar gelagert ist. Auch andere Ausgestaltungen sind jedoch möglich. Das verschiebbare Element kann vollständig in einem Innenraum des Behälters aufgenommen sein und beispielsweise vollständig von einer Behälterwand umgeben sein und/oder kann auch beispielsweise einen Innenraum des Behälters vollständig oder teilweise gegenüber einem Außenraum abschließen und/oder kann ganz oder teilweise als Verschlusselement des Behälters ausgestaltet sein.

Beispielsweise kann der Behälter mindestens eine Behälterwand umfassen, welche beispielsweise aus mindestens einem Kunststoffmaterial und/oder aus mindestens einem Glasmaterial hergestellt sein kann. Beispielsweise kann der Behälter ein röhrenförmiger Behälter sein, wobei der röhrenförmige Behälter vorzugsweise an einem Ende durch die Behälterwand selbst verschlossen ist und an einem anderen Ende und/oder in ihrem Inneren durch das verschiebbare Element, beispielsweise das Verschlusselement, verschlossen ist oder verschlossen werden kann. Alternativ oder zusätzlich zu einem Verschluss durch das verschiebbare Element kann der Behälter auch ganz oder teilweise durch mindestens ein von dem verschiebbaren Element getrennt ausgebildetes Verschlusselement verschlossen sein, beispielsweise durch eine Kappe, beispielsweise eine Bördelkappe, und/oder ein Septum.

Das verschiebbare Element kann beispielsweise ganz oder teilweise als Stopfen ausgestaltet sein, beispielsweise als Kunststoffstopfen. Insbesondere kann das verschiebbare Element perforierbar ausgestaltet sein, sodass dieses mindestens eine perforierbare Element perforiert werden kann, um eine Fluidverbindung zu dem Innenraum des Behälters herzustellen. Alternativ oder zusätzlich kann jedoch auch mindestens ein von dem verschiebbaren Element getrennt ausgebildetes perforierbares Element vorgesehen sein, beispielsweise mindestens ein Septum, welches perforiert werden kann, um die Fluidverbindung zu dem Innenraum des Behälters herzustellen.

Das verschiebbare Element ist relativ zu dem Behälter beweglich gelagert. Beispielsweise kann das verschiebbare Element linear verschiebbar zu mindestens einer Behälterwand des Behälters gelagert sein, beispielsweise indem das verschiebbare Element beweglich ist und insbesondere einen verschiebbar in dem Behälter aufnehmbaren Stopfen umfasst.

Diese bewegliche Lagerung ist derart ausgestaltet, dass eine relative Positionierung des verschiebbaren Elements zu dem Behälter einen zur Aufnahme des fluiden Mediums zur Verfügung stehenden Innenraum des Behälters bestimmt. Beispielsweise kann der Innenraum durch die Behälterwand des Behälters und das verschiebbare Element begrenzt sein, vorzugsweise ausschließlich durch diese Elemente. So kann das fluide Medium beispielsweise, solange dieses in dem Innenraum des Behälters aufgenommen ist, ausschließlich mit der Behälterwand des Behälters und dem verschiebbaren Element, beispielsweise dem verschiebbar gelagerten Stopfen, in Berührung kommen.

Die Medikationsvorrichtung umfasst weiterhin mindestens ein Messelement, welches eingerichtet ist, um die relative Positionierung des verschiebbaren Elements zu dem Behälter zu erfassen. Unter einer relativen Positionierung des verschiebbaren Elements ist allgemein eine Relativposition und/oder relative Orientierung zwischen dem verschiebbaren Element und dem Behälter oder mindestens einem von dem verschiebbaren Element verschiedenen Teil des Behälters zu verstehen, , beispielsweise einer Behälterwand, wobei diese Relativposition und/oder relative Orientierung für die Dimensionierung des zur Aufnahme des fluiden Mediums zur Verfügung stehenden Innenraums des Behälters charakteristisch oder bestimmend ist. So kann es sich dabei um eine Absolutposition des verschiebbaren Elements in dem Behälter handeln, beispielsweise um eine Absolutposition einer Grenzfläche zwischen einem Stopfen und dem fluiden Medium in dem Behälter. Auch andere Ausgestaltungen sind jedoch möglich, beispielsweise abhängig von der Art der Ausgestaltung des verschiebbaren Elements.

Unter einem Messelement ist dementsprechend eine beliebige Vorrichtung zu verstehen, welche eingerichtet ist, um mittels mindestens einer Messmethode die relative Positionierung zu erfassen. Insbesondere kann das Messelement eingerichtet sein, um die relative Positionierung direkt zu erfassen. Unter einer direkten Erfassung der relativen Positionierung des verschiebbaren Elements zu dem Behälter wird eine Erfassung verstanden, bei welcher eine Grenzfläche zwischen dem verschiebbaren Element und dem fluiden Medium in ihrer Positionierung erfasst wird, beispielsweise in einem Koordinatensystem, in welchem der Behälter ruht. Insbesondere kann eine Absolutposition und/oder eine absolute Orientierung in diesem Koordinatensystem erfasst werden.

Die Medikationsvorrichtung umfasst weiterhin mindestens ein Stellglied zur Beeinflussung einer Abgabe des fluiden Mediums über mindestens eine Fluidverbindung zu dem Innenraum. Unter einer Fluidverbindung ist allgemein im Rahmen der vorliegenden Erfindung eine Vorrichtung zu verstehen, welche mindestens einen fluidischen Leiter umfasst, über welchen das fluide Medium aus dem Innenraum heraus in einen den Behälter direkt oder indirekt umgebenden Außenraum transferiert werden kann oder umgekehrt. Insbesondere kann die Fluidverbindung mindestens eine Schlauchverbindung umfassen, insbesondere mindestens eine Schlauchverbindung mit mindestens einem flexiblen Schlauch. Auch andere Ausgestaltungen sind jedoch grundsätzlich möglich.

Unter einem Stellglied wird allgemein im Rahmen der vorliegenden Erfindung ein Element verstanden, welches eingerichtet ist, um die Abgabe des fluiden Mediums zu beeinflussen, also beispielsweise wahlweise zu ermöglichen und zu unterbrechen und/oder wahlweise in unterschiedlichem Maße zu ermöglichen. Dabei kann das Stellglied ganz oder teilweise als passives Stellglied ausgestaltet sein, also als Stellglied, welches den eigentlichen Fluidtransfer selbst nicht fördert, sondern ausschließlich beeinflusst. Dementsprechend kann, wie unten noch näher ausgeführt wird, dass Stellglied mindestens ein Ventil umfassen, welches selbst den Fluidtransfer nicht antreibt, sondern lediglich beeinflusst. Zum eigentlichen Antrieb des Transfers kann beispielsweise, wie unten noch näher ausgeführt wird, das fluide Medium in dem Behälter druckbeaufschlagt sein, beispielsweise durch mindestens eine Energieeinheit, und/oder es kann mindestens ein Antrieb und/oder mindestens eine Pumpe vorgesehen sein, um das fluide Medium aus dem Behälter zu verdrängen und/oder aus dem Behälter zu saugen, um die Abgabe des fluiden Mediums zu fördern. Alternativ oder zusätzlich kann das Stellglied jedoch auch selbst ganz oder teilweise als aktives Stellglied ausgestaltet sein, um den Transfer des fluiden Mediums aus dem Innenraum heraus, also die Abgabe des fluiden Mediums, anzutreiben.

Unter einer Abgabe des fluiden Mediums wird also allgemein im Rahmen der vorliegenden Erfindung ein Prozess verstanden, bei welchem das fluide Medium aus dem Innenraum heraustransferiert wird. Diese Abgabe kann beispielsweise in einen Außenraum erfolgen, welcher von dem Innenraum getrennt ausgestaltet sein kann, und/oder in eine andere Vorrichtung hinein, beispielsweise in ein Gefäß. Auch andere Ausgestaltungen sind möglich.

Weiterhin wird vorgeschlagen, dass das Messelement und das Stellglied derart zusammenwirken, dass die Abgabe des fluiden Mediums durch die von dem Messelement erfasste relative Positionierung beeinflusst wird. Unter einer Beeinflussung der Abgabe des fluiden Mediums ist dabei allgemein ein Vorgang zu verstehen, bei welchem die Art und Weise der Abgabe von der von dem Messelement erfassten relativen Positionierung abhängig ist. So kann die Abgabe insbesondere derart ausgestaltet sein, dass mindestens zwei unterschiedliche relative Positionierungen existieren, welche von dem Messelement erfasst werden können und bei welchen die Abgabe des fluiden Mediums unterschiedlich ausgestaltet ist. Die Abgaben können sich insbesondere hinsichtlich einer Abgaberate und/oder einer Abgabemenge unterscheiden. Allgemein sei darauf hingewiesen, dass die Abgabe kontinuierlich oder auch diskontinuierlich erfolgen kann, sodass beispielsweise ein kontinuierlieher Massenstrom und/oder kontinuierlicher Volumenstrom beeinflusst werden kann und/oder dass eine diskontinuierliche Abgabe, beispielsweise eine schubweise Abgabe, hinsichtlich einer Gesamtmenge und/oder hinsichtlich einer Abgaberate beeinflusst wird. Bei einer diskontinuierlichen Abgabe kann beispielsweise eine Beeinflussung von Zeitdauern von Abgabephasen erfolgen, und/oder es kann ein Verhältnis zwischen Abgabephasen und Phasen, in welchen keine Abgabe des fluiden Mediums erfolgt, beeinflusst werden.

Das Messelement kann insbesondere eingerichtet sein, um mindestens ein Messsignal entsprechend der relativen Positionierung zu erzeugen. Dieses mindestens eine Messsignal kann beispielsweise ein elektrisches Messsignal und/oder ein optisches Messsignal sein. Unter einem Messsignal ist allgemein ein Signal zu versehen, welches drahtgebunden oder auch drahtlos mindestens eine Information trägt. Das Messsignal und/oder die Information können analog oder auch digital ausgestaltet sein. Das Messsignal kann insbesondere eine maschinenlesbare Information über die relative Positionierung beinhalten.

Die Medikationsvorrichtung kann insbesondere mindestens eine Steuerung aufweisen, wobei die Steuerung eingerichtet ist, um das Stellglied mittels des Messsignals zu steuern und/oder zu regeln. So kann allgemein die Abgabe des fluiden Mediums durch die von dem Messelement erfasste Positionierung automatisch beeinflusst werden, beispielsweise mittels der vorgeschlagenen Steuerung. Diese Steuerung kann beispielsweise eine Elektronik und/oder eine Datenverarbeitungsvorrichtung umfassen, welche zur Steuerung und/oder zur Regelung des Messsignals eingerichtet ist. Unter dem Vorgang des Steuerns, welcher von der Steuerung durchgeführt werden kann, wird allgemein ein Vorgang verstanden, bei welchem eine zu steuernde Größe, beispielsweise über mindestens ein Stellglied, welches mindestens eine Steuerstrecke beeinflussende Stellgröße erzeugt, auf mindestens eine Führungsgröße eingestellt wird. Unter dem Vorgang des Regelns, welcher alternativ oder zusätzlich von der Steuerung durchgeführt werden kann, ist allgemein im Rahmen der vorliegenden Erfindung ein Vorgang zu verstehen, bei welchem ein Istwert einer Regelgröße erfasst wird und bei welchem, abhängig von einer Abweichung des Istwerts von einem Sollwert, mindestens eine Korrekturmaßnahme ergriffen wird, beispielsweise indem über mindestens ein Stellglied wiederum eine Stellgröße erzeugt wird, die auf eine Regelstrecke einwirkt.

So kann insbesondere die Medikationsvorrichtung derart eingerichtet sein, dass mittels des Messsignals des Messelements das Stellglied gesteuert und/oder geregelt wird. Beispielsweise kann die Medikationsvorrichtung derart eingerichtet sein, dass mindestens eine Gesamtmenge einer Abgabe des fluiden Mediums und/oder eine Abgaberate des fluiden Mediums vorgegeben werden, welche als Führungsgröße verwendet werden, welche ihrerseits als Führungsgröße für die Steuerung und/oder als Sollwert für die Regelung verwendet werden können. Die Vorgabe kann durch die Medikationsvorrichtung selbst erfolgen, kann durch eine andere Vorrichtung erfolgen, oder kann auch beispielsweise durch einen Benutzer erfolgen, beispielsweise indem der Benutzer diese Vorgabe mittels eines oder mehrerer Eingabeelemente der Medikationsvorrichtung vorgibt. Die Medikationsvorrichtung kann insbesondere eingerichtet sein, um eine abgegebene Absolutmenge und/oder eine Abgaberate des fluiden Mediums durch die von dem Messelement erfasste relative Positionierung zu beeinflussen, insbesondere einzustellen, zu steuern oder zu regeln.

Wie oben ausgeführt, kann das Messelement insbesondere eingerichtet sein, um die relative Positionierung des verschiebbaren Elements zu dem Behälter direkt zu erfassen. Das verschiebbare Element kann beispielsweise einen relativ zu dem Behälter verschiebbaren Stopfen umfassen. Das Messelement kann beispielsweise eingerichtet sein, um eine Verschiebung des Stopfens in dem Behälter, insbesondere eine lineare Verschiebung, zu erfassen.

Das Messelement kann grundsätzlich auf verschiedene Weisen eingerichtet sein, welche einzeln oder auch in beliebiger Kombination realisierbar sind. So kann das Messelement insbesondere ausgewählt sein aus der Gruppe bestehend aus einem mechanischen Messelement, einem magnetischen Messelement, einem elektronischen Messelement, einem induktiven Messelement, einem kapazitiven Messelement, einem resistiven Messelement, einem optischem Messelement und einem Ultraschall-Messelement. Auch Kombinationen der genannten Gruppe und/oder Kombinationen der beschriebenen Prinzipien mit einem oder mehreren anderen Prinzipien sind einsetzbar.

Wie oben ausgeführt, kann das Messelement insbesondere eingerichtet sein, um mindestens eine Position mindestens einer Grenzfläche zwischen dem verschiebbaren Element und dem fluiden Medium zu erfassen. Dies kann beispielsweise dadurch erfolgen, dass direkt eine Verschiebung zwischen dem verschiebbaren Element und dem fluiden Medium umfasst wird.

Das Messelement kann insbesondere eingerichtet sein, um die relative Positionierung mit einer Genauigkeit von mindestens 0,05 mm, insbesondere von 0,01 mm zu erfassen.

Weitere mögliche Ausgestaltungen betreffen die Fluidverbindung. So kann diese Fluidverbindung insbesondere das verschiebbare Element und vorzugsweise den Stopfen durchdringen. Beispielsweise kann das verschiebbare Element einen perforierbaren Stopfen umfassen, beispielsweise einen Stopfen aus mindestens einem Kunststoffmaterial und besonders bevorzugt aus mindestens einem Elastomermaterial. Die Fluidverbindung kann beispielsweise mindestens eine Kanüle umfassen, welche den perforierbaren Stopfen durchdringt. Auch andere Ausgestaltungen des verschiebbaren Elements als eine Ausgestaltung als perforierbares Verschlusselement sind jedoch grundsätzlich möglich. Weitere mögliche Ausgestaltungen betreffen die bewegliche Lagerung des verschiebbaren Elements relativ zu dem Behälter. Erfindungsgemäß weist die Medikationsvorrichtung mindestens ein Gehäuse auf. Unter einem Gehäuse ist allgemein eine Vorrichtung zu verstehen, welche die Medikationsvorrichtung nach außen vor äußeren mechanischen Einflüssen schützt. So kann das Gehäuse beispielsweise formstabil ausgestaltet sein, also derart, dass sich dieses - zumindest unter Einwirkung seiner eigenen Gewichtskraft - nicht verformt. Das Gehäuse kann beispielsweise ganz oder teilweise als Kunststoffgehäuse oder als Metallgehäuse ausgestaltet sein. Der Behälter kann insbesondere beweglich in dem Gehäuse aufgenommen sein, und das verschiebbare Element kann relativ zu dem Gehäuse fixiert sein, beispielsweise indem das verschiebbare Element fest mit dem Gehäuse verbunden ist oder sogar Bestandteil des Gehäuses bildet. Auch eine inverse Ausgestaltung ist jedoch grundsätzlich möglich, bei welcher der Behälter fest in den Gehäuse aufgenommen ist und das verschiebbare Element beweglich in dem Gehäuse gelagert ist, oder eine Kombination, bei welcher sowohl der Behälter als auch das verschiebbare Element relativ zu dem Gehäuse beweglich gelagert sind. Erfindungsgemäß ist die
Ausgestaltung, bei welcher der Behälter beweglich in dem Gehäuse aufgenommen ist und bei welcher das verschiebbare Element relativ zu dem Gehäuse fixiert ist, wobei das Messelement eingerichtet ist, um eine relative Positionierung des Behälters in dem Gehäuse zu erfassen. Aus dieser relativen Positionierung des Behälters in dem Gehäuse kann dann wiederum, da das verschiebbare Element relativ zu dem Gehäuse fixiert ist, auf eine relative Positionierung zwischen dem Behälter und dem verschiebbaren Element geschlossen werden.

Wie oben ausgeführt, sind verschiedene Arten der Ausgestaltung des fluiden Mediums möglich. So ist grundsätzlich eine Ausgestaltung möglich, bei welcher das fluide Medium aus dem Innenraum des Behälters gesaugt wird. Alternativ oder zusätzlich ist jedoch auch eine Ausgestaltung möglich, bei welcher durch eine Druckbeaufschlagung mit einem Überdruck das fluide Medium aus dem Innenraum heraus befördert wird. Dieser Überdruck kann beispielsweise durch eine mechanische oder auch hydraulische und/oder pneumatische Druckbeaufschlagung erfolgen. Beispielsweise kann die Druckbeaufschlagung mit dem Überdruck dadurch erfolgen, dass das verschiebbare Element in den Innenraum hinein bewegt wird und/oder dass auf andere Weise durch eine Veränderung der relativen Positionierung und/oder relativen Orientierung zwischen dem verschiebbaren Element und mindestens einem weiteren Teil des Behälters, beispielsweise eine Behälterwand, der zur Aufnahme des fluiden Mediums zur Verfügung stehenden Innenraum des Behälters verkleinert wird.

Die Medikationsvorrichtung kann beispielsweise eingerichtet sein, um das verschiebbare Element und/oder den Behälter derart mit einer Kraft zu beaufschlagen, dass ein Überdruck auf das fluide Medium ausgeübt wird und/oder dass das fluide Medium unter einem erhöhten Druck steht, beispielsweise unter einem gegenüber dem Umgebungsdruck erhöhten Druck. Insbesondere kann die Medikationsvorrichtung eingerichtet sein, um das verschiebbare Element gegen ein Widerlager zu pressen, sodass das verschiebbare Element in den Innenraum hineingedrückt wird. Unter einem Hineindrücken ist dabei ein Vorgang zu verstehen, bei welchem durch eine Veränderung der Positionierung des verschiebbaren Elements relativ zu dem Behälter, unabhängig davon ob nunmehr der Behälter und/oder das verschiebbare Element bewegt werden, ein Volumen des Innenraums verringert wird. Das Widerlager kann dabei beispielsweise Bestandteil eines Gehäuses sein. Besonders bevorzugt ist es jedoch, wenn das Widerlager ganz oder teilweise Bestandteil des Transport- und Transferbehältnisses ist, beispielsweise eines Behältergehäuses des Transport-und Transferbehältnisses, welches nicht notwendigerweise Bestandteil des Gehäuses der Medikationsvorrichtung sein muss und welches vorzugsweise unabhängig von dem Gehäuse der Medikationsvorrichtung ausgestaltet und gehandhabt werden kann. So kann beispielsweise ein Behältergehäuse des Transport- und Transferbehältnisses vorgesehen sein, welches ein Widerlager aufweist, auf welchem das verschiebbare Element aufsitzt, wobei der Behälter des Transport- und Transferbehältnisses relativ zu dem Widerlager verschiebbar ist, sodass das verschiebbare Element mittels des Widerlagers in den Innenraum hineingedrückt wird.

Um das verschiebbare Element gegen das Widerlager zu pressen, kann die Medikationsvorrichtung, insbesondere das Transport- und Transferbehältnis, beispielsweise mindestens ein Federelement aufweisen. Beispielsweise kann dieses Federelement Bestandteil des Transport- und Transferbehältnisses sein. So kann das Transport- und Transferbehältnis, wie oben ausgeführt, insbesondere mindestens ein Behältergehäuse aufweisen, wobei der Behälter innerhalb des Behältergehäuses aufgenommen ist und wobei zusätzlich das mindestens eine Federelement in dem Behältergehäuse aufgenommen ist, sodass beispielsweise der Behälter derart mit einer Kraft beaufschlagt wird, dass das verschiebbare Element gegen das Widerlager gepresst und in den Innenraum hineingedrückt wird.

Die Fluidverbindung kann insbesondere durch das Widerlager hindurch erfolgen. So kann das Widerlager beispielsweise zylindrisch ausgestaltet sein, beispielsweise als Hohlzylinder, wobei sich beispielsweise eine Kanüle der Fluidverbindung und/oder ein Schlauch der Fluidverbindung durch das Widerlager hindurch und vorzugsweise auch durch das verschiebbare Element hindurch in den Innenraum erstreckt.

Die Medikationsvorrichtung kann insbesondere derart ausgestaltet sein, dass diese mindestens eine Energieeinheit aufweist. Die Energieeinheit ist eingerichtet, um das fluide Medium in dem Behälter mit einem Druck zu beaufschlagen. Somit ist unter einer Energieeinheit im Rahmen der vorliegenden Erfindung allgemein eine Vorrichtung zu verstehen, welche eingerichtet ist, um das fluide Medium mit dem Überdruck zu beaufschlagen. Die Energieeinheit kann insbesondere mindestens einen Energiespeicher und/oder mindestens einen Aktor umfassen, welcher für die Druckbeaufschlagung eingerichtet ist. Insbesondere kann die Energieeinheit mindestens ein Federelement aufweisen. Die Energieeinheit kann insbesondere mindestens einen Energiespeicher aufweisen, insbesondere einen mechanischen Energiespeicher und besonders bevorzugt mindestens ein Federelement. Die Energieeinheit kann eingerichtet sein, um die Druckbeaufschlagung mit dem Überdruck für eine Zeitdauer von mindestens einem Monat aufrechtzuerhalten, vorzugsweise für mindestens sechs Monate und besonders bevorzugt für mindestens ein Jahr oder sogarfür die gesamte Lagerlebensdauer oder bis zum Ablauf des Haltbarkeitsdatums der Medikationsvorrichtung und/oder des Transport- und Transferbehältnisses, sodass der Überdruck beispielsweise von einem Anfangswert innerhalb der genannten Zeitdauer auf nicht weniger als 80% abgesunken ist.

Die Energieeinheit kann insbesondere ganz oder teilweise Bestandteil des Transport- und Transferbehältnisses sein. Wie oben ausgeführt, kann das Transport- und Transferbehältnis beispielsweise mindestens ein Behältergehäuse aufweisen, wobei der Behälter beispielsweise in dem Behältergehäuse aufgenommen ist. Die Energieeinheit kann insbesondere ganz oder teilweise in einem Zwischenraum zwischen dem Behältergehäuse und dem Behälter aufgenommen sein, beispielsweise in einem Ringspalt zwischen dem Behältergehäuse und dem Behälter. Insbesondere kann das Federelement in diesem Zwischenraum gelagert sein, beispielsweise in Form einer Schraubendruckfeder oder Spiralfeder.

Das Transport- und Transferbehältnis kann insbesondere in einem druckbeaufschlagten Zustand, in welchem das fluide Medium in dem Behälter mit dem Überdruck beaufschlagt ist, unabhängig von weiteren Komponenten der Medikationsvorrichtung gehandhabt werden. So kann beispielsweise das Transport- und Transferbehältnis bereits herstellerseitig in einem druckbeaufschlagten Zustand geliefert werden, um dann beispielsweise in die Medikationsvorrichtung integriert zu werden, sodass seitens der Medikationsvorrichtung für die Druckbeaufschlagung keine weiteren Vorrichtungen erforderlich sein müssen.

Weitere mögliche Ausgestaltungen betreffen das mindestens eine Stellglied. So kann dieses Stellglied insbesondere mindestens ein Ventil aufweisen. Dieses Ventil kann eingerichtet sein, um einen Durchfluss des fluiden Mediums durch die Fluidverbindung zu beeinflussen. Diese Beeinflussung kann derart erfolgen, dass mindestens zwei verschiedene Ventilstellungen vorgesehen sein können, in welchen ein unterschiedlicher Durchfluss des fluiden Mediums durch die Fluidverbindung erfolgt, beispielsweise mit unterschiedlichen Abgabemengen und/oder unterschiedlichen Abgaberaten. Alternativ oder zusätzlich kann das Ventil jedoch auch derart ausgestaltet sein, dass ein Durchfluss des fluiden Mediums durch die Fluidverbindung wahlweise unterbrochen wird oder freigegeben wird.

Die Fluidverbindung kann insbesondere, wie oben ausgeführt, mindestens eine Schlauchverbindung aufweisen. Unter einer Schlauchverbindung ist allgemein eine Vorrichtung zu verstehen, welche mindestens ein röhrenförmiges, flexibles und/oder verformbares Element aufweist. Beispielsweise kann die Schlauchverbindung mindestens einen Kunststoffschlauch, vorzugsweise mindestens einen Elastomerschlauch und besonders bevorzugt mindestens einen Silikonschlauch aufweisen.

Das Ventil kann insbesondere mindestens ein Ventilglied aufweisen. Dieses Ventilglied kann insbesondere eingerichtet sein, um die Schlauchverbindung in mindestens einem Quetschbereich zu quetschen. Dieses Quetschen kann vollständig oder auch teilweise erfolgen, sodass bei dem Quetschen beispielsweise ein Durchfluss des fluiden Mediums durch den Quetschbereich gedrosselt oder vollständig unterbrochen wird. Insbesondere kann das mindestens eine Ventil mindestens zwei Ventilglieder aufweisen, wobei diese mindestens zwei Ventilglieder Bestandteil ein und desselben Ventils sein können oder auch Bestandteile verschiedener Ventile sein können. Dementsprechend kann die Schlauchverbindung durch ein einziges Ventil oder auch durch mehrere Ventile gequetscht werden. Die Quetschbereiche können statisch und in fester Positionierung vorgegeben sein, können jedoch auch beweglich angeordnet sein, sodass sich beispielsweise die Quetschbereiche relativ zu der Schlauchverbindung auch bewegen können und beispielsweise wandem können. Auf diese Weise kann das Ventil auch beispielsweise ganz oder teilweise eine Pumpenfunktion erfüllen, beispielsweise eine Funktion einer Peristaltikpumpe. Dementsprechend kann das mindestens eine Stellglied auch beispielsweise eine Kombination mindestens eines Ventilglieds und mindestens einer Pumpe, beispielsweise einer Peristaltikpumpe, umfassen. Das Ventilglied kann beispielsweise mindestens ein Quetschelement aufweisen, beispielsweise ein Ende des Ventilglieds, welches einen Druck auf die Schlauchverbindung ausübt, gegebenenfalls in Kombination mit mindestens einem Widerlager, sodass die Schlauchverbindung gequetscht und verformt wird.

Die Schlauchverbindung kann insbesondere zumindest partiell eine Wandstärke aufweisen, welche einen Innendurchmesser der Schlauchverbindung überschreitet. Diese Ausgestaltung ist vorteilhaft, da auf diese Weise beispielsweise mindestens eine Stellung des mindestens einen Ventilgliedes existieren kann, in welcher die Schlauchverbindung bereits gequetscht ist und in welcher ein Durchfluss durch den Quetschbereich verhindert wird. Ausgehend von dieser Stellung kann beispielsweise ein weiteres Quetschen erfolgen, bei welchem, da der Innendurchmesser nunmehr bereits auf 0 reduziert ist, lediglich ein Wandbereich der Schlauchverbindung weiter gequetscht wird. Diese Ausgestaltung ist besonders bevorzugt, wenn ein Ventil mehrere Ventilglieder aufweist, die mechanisch miteinander gekoppelt sind, beispielsweise in Form einer Ventilwippe. Eine Wand der Schlauchverbindung kann dementsprechend als Reservoir für eine überschüssige Quetschung und eine überschüssige Verformung der Schlauchverbindung dienen. Der Begriff des Innendurchmessers ist dabei allgemein weit zu fassen. Weist die Schlauchverbindung einen nicht-kreisförmigen Querschnitt auf, so kann unter einem Innendurchmesser allgemein ein Äquivalentdurchmesser verstanden werden.

Das Ventil kann insbesondere mindestens zwei geschlossene Ventilstellungen aufweisen, wobei ein Durchfluss des fluiden Mediums unterbrochen ist, wenn das Ventil in einer der geschlossenen Ventilstellungen ist. Unter einer Ventilstellung wird dabei allgemein ein Zustand des Ventils verstanden. Dieser Zustand kann sich auf ein einziges Ventilglied beziehen, welches mindestens zwei Stellungen einnehmen kann, oder kann sich auch auf mehrere Ventilglieder beziehen, welche unterschiedliche Ventilstellungen einnehmen können. Die geschlossenen Ventilstellungen können insbesondere Quetschpositionen sein, wobei in den Quetschpositionen die Fluidverbindung, insbesondere eine Schlauchverbindung der Fluidverbindung, gequetscht wird.

Die geschlossenen Ventilstellungen können insbesondere Endstellungen einer Bewegung eines Ventilglieds des Ventils umfassen, wobei sich das Ventilglied beispielsweise zwischen den Ventilstellungen bewegen kann. Das Ventilglied kann insbesondere ein um eine Achse drehbar gelagertes Ventilglied aufweisen, welches über mindestens zwei Druckelemente, beispielsweise einander gegenüber liegende Enden des Ventilglieds, insbesondere zwei Quetschelemente, die Fluidverbindung unterbrechen kann. So kann das um die Achse drehbar gelagerte Ventilglied beispielsweise als Wippe ausgestaltet sein, wobei die mindestens zwei Druckelemente mindestens zwei auf unterschiedlichen Armen der Ventilwippe angeordnete Abschnitte bilden, beispielsweise Endabschnitte.

Das Ventil kann insbesondere derart ausgestaltet sein, dass sich dieses in einer Ruhestellung, in welcher keine Kraft auf ein Ventilglied des Ventils ausgeübt wird, in einer geschlossenen Ventilstellung befindet, in welcher der Durchfluss des fluiden Mediums durch die Fluidverbindung unterbrochen ist. Insbesondere kann die Fluidverbindung mindestens eine Schlauchverbindung aufweisen, wobei in der Ruhestellung das Ventilglied die Schlauchverbindung in mindestens zwei Quetschpositionen quetscht und dadurch die Fluidverbindung in den mindestens zwei Quetschpositionen unterbricht. Ausgehend von dieser Ruhestellung kann sich beispielsweise das Ventilglied in zwei Richtungen bewegen, wobei, je nach Richtung, die erste Quetschposition für einen Durchfluss des fluiden Mediums freigegeben wird und die zweite Quetschposition weiter gequetscht und damit geschlossen gehalten wird, oder umgekehrt.

Das Ventil kann insbesondere, wie oben ausgeführt, mindestens ein Ventilglied aufweisen. Dieses Ventilglied kann insbesondere in mindestens zwei statische Ventilpositionen einstellbar sein. Unter einer statischen Ventilposition ist dabei eine Ventilposition zu verstehen, bei welcher das Ventilglied während eines routinemäßigen Betriebs der Medikationsvorrichtung verharrt. Das Ventil kann insbesondere derart ausgestaltet sein, dass in allen vorgesehenen statischen Ventilpositionen ein Durchfluss des fluiden Mediums durch die Fluidverbindung unterbrochen ist, sodass das Ventil insbesondere eigensicher ausgestaltet sein kann. Beispielsweise können mindestens zwei statische Ventilpositionen vorgesehen sein, wobei in jeder dieser statischen Ventilpositionen eine Schlauchverbindung in mindestens einer Quetschposition gequetscht wird. Beispielsweise kann eine erste statische Ventilposition vorgesehen sein, in welcher die Schlauchverbindung in mindestens einer ersten Quetschposition gequetscht wird, und mindestens eine zweite statische Ventilposition, in welcher die Schlauchverbindung in mindestens einer zweiten, von der ersten verschiedenen Quetschposition gequetscht wird, sodass jeweils entweder in der ersten Quetschposition oder in der zweiten Quetschposition eine Unterbrechung des Durchflusses des fluiden Mediums erfolgt. Auf diese Weise kann durch die Eigensicherung gewährleistet werden, dass nie eine Überdosierung des fluiden Mediums erfolgt, beispielsweise indem ein ungehinderter Durchfluss des fluiden Mediums durch das Ventil erfolgt.

Das Ventil kann insbesondere mindestens zwei Ventilstellungen aufweisen, insbesondere mindestens zwei Ventilstellungen mindestens eines Ventilglieds des Ventils, beispielsweise mindestens zwei Ventilstellungen desselben Ventilglieds des Ventils, wobei die Ventilstellungen jeweils derart ausgestaltet sind, dass der Durchfluss des fluiden Mediums jeweils an mindestens einer anderen Stelle der Fluidverbindung unterbrochen ist.

Wie oben ausgeführt, kann das mindestens eine Stellglied ganz oder teilweise passives Stellglied ausgestaltet sein, welches einen Durchfluss des fluiden Mediums beeinflusst, ohne den eigentlichen Transfer des fluiden Mediums aus dem Behälter heraus anzutreiben, beispielsweise in Form mindestens eines Ventils. Alternativ oder zusätzlich kann das mindestens eine Stellglied jedoch ganz oder teilweise als aktives Stellglied ausgestaltet sein und eingerichtet sein, um den Transfer des fluiden Mediums anzutreiben. Letztere Ausgestaltung ist besonders sinnvoll, wenn das Transport- und Transferbehältnis in einem Ruhezustand nicht mit einem Überdruck beaufschlagt ist.

In einer vorteilhaften Ausgestaltung kann das Stellglied daher insbesondere mindestens eine Pumpe aufweisen. Diese Pumpe kann insbesondere eingerichtet sein, um das fluide Medium aus dem Innenraum des Behälters zu saugen und/oder um das fluide Medium durch die Fluidverbindung zu fördern und/oder um das fluide Medium kontrolliert abzugeben.

Diese Pumpe kann auf verschiedene Weisen ausgestaltet sein. Besonders vorteilhaft ist es, wenn die Pumpe mindestens eine Peristaltikpumpe aufweist oder ganz oder teilweise als Peristaltikpumpe ausgestaltet ist. Unter einer Peristaltikpumpe oder peristaltischen Pumpe ist dabei allgemein eine Verdrängerpumpe zu verstehen, bei welcher das fluide Medium durch eine mechanische Verformung der Fluidverbindung oder eines Teils der Fluidverbindung, insbesondere einer Schlauchverbindungs der Fluidverbindung, gefördert wird. Zum Zweck dieser mechanischen Verformung, welche beispielsweise wiederum ein Quetschen der Schlauchverbindung beinhalten kann, kann die Pumpe beispielsweise zwei, drei oder mehr Aktoren aufweisen, welche, beispielsweise in einem vorgegebenen zeitlichen Ablauf, nacheinander die Fluidverbindung verformen, beispielsweise die Schlauchverbindung nacheinander in unterschiedlichen Positionen quetschen. Derartige peristaltische Pumpen eignen sich insbesondere für hochviskose fluide Medien. Beispielsweise werden peristaltische Pumpen, welche auch im Rahmen der vorliegenden Erfindung eingesetzt werden können, in Y.-C. Hsu et al.: Development of peristaltic antithrombogenic micropumps for in vitro and ex vivo blood transportation tests, Microsyst. Technol. (2007) 14:31-41, beschrieben. Auch andere peristaltische Pumpen können jedoch grundsätzlich eingesetzt werden.

Allgemein kann die Medikationsvorrichtung beispielsweise derart ausgestaltet sein, dass das Stellglied mindestens zwei Aktuatoren umfasst, wobei die Fluidverbindung mindestens einen Schlauchabschnitt umfasst, wobei die Aktuatoren eingerichtet sind, um den Schlauchabschnitt in mindestens zwei verschiedenen Quetschbereichen zu quetschen, beispielsweise räumlich und/oder zeitlich versetzt. Die mindestens zwei Aktuatoren können unabhängig voneinander ausgestaltet sein, können jedoch auch elektrisch und/oder mechanisch miteinander verbunden sein, sodass beispielsweise eine zeitliche und/oder räumliche Abfolge des Quetschens durch die Verbindung vorgegeben sein kann. Zwischen den Aktuatoren kann insbesondere in dehnbarer Bereich des Schlauchabschnitts angeordnet sein. Die Aktuatoren können insbesondere mindestens einen einlasseitigen Aktuator und mindestens einen auslassseitigen Aktuator und mindestens einen auslassseitigen Aktuator umfassen. Die Begriffe "einlassseitig" und "auslassseitig" beziehen sich dabei darauf, wie das fluide Medium bei der Abgabe des fluiden Mediums aus der Medikationsvorrichtung durch den Schlauchabschnitt strömt. So ist der einlassseitige Aktuator derjenige der Aktuatoren, welcher von einem Volumenelement des fluiden Mediums zeitlich zuerst passiert wird. Die Medikationsvorrichtung kann insbesondere eingerichtet sein, um bei geöffnetem einlassseitigem Aktuator und geschlossenem auslassseitigem Aktuator fluides Medium unter Druck in den dehnbaren Bereich zwischen den Aktuatoren einzulassen. Unter einem "geschlossenen" Aktuator wird dabei eine Stellung des Aktuators verstanden, in welcher der jeweilige Aktuator den Schlauchabschnitt in dem Quetschbereich quetscht und einen Durchlass durch diesen Quetschbereich verhindert. Bei dem genannten Einlass in den dehnbaren Bereich kann der dehnbare Bereich gedehnt werden. Die Medikationsvorrichtung kann weiterhin eingerichtet sein, um anschließend den einlassseitigen Aktuator zu schließen und den auslassseitigen Aktuator zu öffnen, wobei der dehnbare Bereich entspannt wird und ein Teil des fluiden Mediums aus dem dehnbaren Bereich ausströmt.

Weitere optionale Ausgestaltungen betreffen den Aufbau der Medikationsvorrichtung. So kann diese Medikationsvorrichtung insbesondere modular aufgebaut sein und insbesondere mindestens zwei Module aufweisen, welche, vorzugsweise reversibel, miteinander verbindbar sind. So kann die Medikationsvorrichtung insbesondere mindestens ein Steuermodul mit mindestens einer Steuerung aufweisen, beispielsweise der oben genannten Steuerung, welche eingerichtet ist, um das Stellglied mittels des Messsignals zu steuern und/oder zu regeln. Allgemein kann unter einem Steuermodul jedoch im Rahmen der vorliegenden Ausgestaltung ein beliebiges Modul verstanden werden, welches eine vollständige oder teilweise Steuerung der Medikationsvorrichtung übernehmen kann. Das Transport- und Transferbehältnis kann, in Alleinstellung oder mit einer oder mehreren Komponenten, mindestens ein weiteres Modul der Medikationsvorrichtung bilden. Das Transport-und Transferbehältnis kann insbesondere austauschbar ausgestaltet sein.

Die Medikationsvorrichtung kann weiterhin insbesondere mindestens ein Basismodul aufweisen, mit mindestens einer Basisplatte, wobei das Steuermodul und das Transport- und Transferbehältnis direkt oder indirekt mit der Basisplatte verbindbar sind. Die Basisplatte kann beispielsweise ein Element sein, welches eine ebene oder gekrümmte Auflagefläche aufweist, welche beispielsweise direkt oder indirekt auf eine Hautoberfläche eines Benutzers aufbringbar ist.

Die Medikationsvorrichtung kann weiterhin mindestens ein Fluidikmodul umfassen. Dieses Fluidikmodul kann zumindest einen Teil der Fluidverbindung umfassen. Beispielsweise kann das Fluidikmodul mindestens eine Schlauchverbindung aufweisen. Das Fluidikmodul kann insbesondere als Einwegteil ausgestaltet sein, sodass beispielsweise mit einem Wechsel des Transport- und Transferbehältnisses auch das Fluidikmodul ausgetauscht werden kann.

Das Transport- und Transferbehältnis kann insbesondere derart ausgestaltet sein, dass die Fluidverbindung bei einer Verbindung der Module herstellbar ist. Unter einem Herstellen der Fluidverbindung kann dabei eine Vervollständigung der Fluidverbindung derart verstanden werden, dass eine Abgabe des fluiden Mediums durch die Fluidverbindung aus dem Innenraum heraus ermöglicht wird. Dies kann insbesondere dadurch erfolgen, dass eine Perforation des verschiebbaren Elements erfolgt.

In einem weiteren Aspekt der vorliegenden Erfindung wird ein Verfahren zur dosierten Abgabe eines fluiden Mediums vorgeschlagen. Dieses Verfahren kann insbesondere unter Verwendung einer Medikationsvorrichtung gemäß einer oder mehreren der oben beschriebenen Ausgestaltungen erfolgen, sodass für mögliche Ausgestaltungen des Verfahrens auf die obige Beschreibung verwiesen werden kann. Insbesondere kann ein fluides Therapeutikum und/oder Diagnostikum erfolgen. Das Verfahren kann einerseits für therapeutische und/oder diagnostische Zwecke eingesetzt werden, beispielsweise an einem menschlichen oder tierischen Körper. Alternativ kann das Verfahren jedoch auch derart durchgeführt werden, dass keine therapeutische und keine diagnostische Funktion an einem menschlichen oder tierischen Körper durchgeführt wird. So kann eine Abgabe des fluiden Mediums beispielsweise an eine andere Vorrichtung erfolgen, beispielsweise für eine Analyse oder zum Zweck einer in-vitro-Diagnostik. Das Verfahren kann insbesondere unter Verwendung einer Medikationsvorrichtung gemäß einer oder mehreren der Ausgestaltungen gemäß der vorliegenden Erfindung durchgeführt werden. Auch andere Ausgestaltungen sind jedoch grundsätzlich möglich.

Das Verfahren umfasst die folgenden Verfahrensschritte, welche vorzugsweise, jedoch nicht notwendigerweise, in der dargestellten Reihenfolge durchgeführt werden. Einzelne oder mehrere Verfahrensschritte können auch einzeln, gruppenweise oder insgesamt wiederholt durchgeführt werden. Weiterhin können auch zwei oder mehr Verfahrensschritte zeitlich parallel oder zeitlich überlappend durchgeführt werden. Weiterhin kann das Verfahren zusätzliche, nicht genannte Verfahrensschritte umfassen.

Bei dem Verfahren wird mindestens ein Transport- und Transferbehältnis verwendet, wobei das Transport- und Transferbehältnis mindestens einen Behälter zur Aufnahme des fluiden Mediums aufweist. Der Behälter weist mindestens ein verschiebbares Element auf. Beispielsweise kann der Behälter durch ein verschiebbares Element in Form eines Verschlusselements verschlossen werden. Das verschiebbare Element wird relativ zu dem Behälter beweglich gelagert. Eine relative Positionierung des verschiebbaren Elements zu dem Behälter bestimmt einen zur Aufnahme des fluiden Mediums zur Verfügung stehenden Innenraum des Behälters.

Weiterhin wird mittels mindestens eines Messelements die relative Positionierung des verschiebbaren Elements zu dem Behälter erfasst.

Weiterhin wird bei dem vorgeschlagenen Verfahren mindestens ein Stellglied zur Beeinflussung einer Abgabe des fluiden Mediums über mindestens eine Fluidverbindung zu dem Innenraum verwendet.

Das Messelement und das Stellglied wirken bei dem erfindungsgemäßen Verfahren derart zusammen, dass die Abgabe des fluiden Mediums durch die von dem Messelement erfasste relative Positionierung beeinflusst wird.

Der Behälter kann bei der Durchführung des Verfahrens bereits mit dem fluiden Medium gefüllt sein. Alternativ kann ein Befüllen des Behälters jedoch auch Bestandteil des Verfahrens sein. Das Befüllen des Behälters kann herstellerseitig erfolgen. Alternativ kann der Behälter jedoch auch ganz oder teilweise von einem Benutzer befüllt werden, beispielsweise im Rahmen eines bestimmungsgemäßen Verwendungsprozesses. Dieses Befüllen kann beispielsweise durch das verschiebbare Element hindurch erfolgen, beispielsweise mittels mindestens einer Spritze.

Für weitere mögliche Ausgestaltungen kann insbesondere auf die obige Beschreibung der Medikationsvorrichtung verwiesen werden.

In einem weiteren Aspekt der vorliegenden Erfindung wird eine Verwendung der Medikationsvorrichtung gemäß der vorliegenden Erfindung in eine oder mehreren der vorangehend oder nachfolgend noch näher beschriebenen Ausgestaltungen vorgeschlagen. Diese Verwendung kann wiederum zu therapeutischen und/oder diagnostischen Zwecken an einem menschlichen Körper erfolgen. Alternativ oder zusätzlich kann die Verwendung jedoch auch ausschließlich zu nicht-therapeutischen und nicht-diagnostischen Zwecken erfolgen und/oder ausschließlich zu therapeutischen und/oder diagnostischen Zwecken, welche nicht an einem menschlichen oder tierischen Körper durchgeführt werden. Die Verwendung sieht vor, dass die Medikationsvorrichtung zur Dosierung eines Therapeutikums und/oder Diagnostikums erfolgt, insbesondere zur Dosierung von Insulin. Die Dosierung kann, wie oben ausgeführt, in einem menschlichen oder tierischen Körper hinein erfolgen oder kann auch in eine unabhängige Vorrichtung erfolgen. Verschiedene Ausgestaltungen sind möglich.

Die Medikationsvorrichtung, das Verfahren und die Verwendung gemäß der vorliegenden Erfindung weisen gegenüber bekannten Medikationsvorrichtungen, bekannten Verfahren und bekannten Verwendungen eine Vielzahl von Vorteilen auf. So lässt sich insbesondere ein neuartiges Pumpensystem, insbesondere eine Medikationspumpe, realisieren.

In einer möglichen Ausgestaltung kann, wie oben beschrieben, das fluide Medium, beispielsweise die Injektionsflüssigkeit, permanent unter einem Überdruck gehalten werden. Dabei kann beispielsweise mittels der oben beschriebenen Energieeinheit, beispielsweise einem Energiespeicher und vorzugsweise einer Druckfeder, ein Überdruck auf das fluide Medium ausgeübt werden, beispielsweise konstant. Aufgrund einer erhöhten Gaslöslichkeit kann dabei ein blasenfreies System realisiert werden.

Weiterhin ist als Vorteil zu vermerken, dass ein feinmechanischer Vortrieb entfallen kann. Insbesondere kann die Medikationsvorrichtung ohne ein mittels eines Antriebs angetriebenen Kolben ausgestaltet werden. Dementsprechend muss keine elektrische Energie für einen derartigen feinmechanischen Vortrieb bereitgestellt werden, wodurch beispielsweise ein elektrischer Energiespeicher entfallen kann.

Weiterhin lässt sich die Medikationsvorrichtung insgesamt kompakt und mit wenigen Komponenten ausgestalten. Beispielsweise kann die Medikationsvorrichtung, wie oben ausgeführt, mindestens ein Transport- und Transferbehältnis beinhalten, welches lediglich aus zwei Bauteilen besteht, nämlich dem Behälter und dem verschiebbaren Element. So kann beispielsweise ein verschieblicher und optional penetrierbarer Stopfen als verschiebbares Element fest positioniert werden, und als Behälter kann eine einseitig geöffnete Röhre verwendet werden, welche sich beim Entleeren des Behälters im Gehäuse verschiebt.

Weiterhin kann, mittels des Messelements, eine tatsächliche relative Positionierung des verschiebbaren Elements zu dem Behälter erfasst werden. Beispielsweise kann eine tatsächliche Verschiebung des Behälters und/oder des verschiebbaren Elements und/oder eines Kolbens gemessen werden. Dies kann beispielsweise über das beschriebene Messelement geschehen. Das Messelement kann einen oder mehrere geeignete Sensoren umfassen, welche beispielsweise mechanisch, optisch oder auf andere Weise ausgestaltet sein können. Mittels dieses mindestens einen Messelements kann beispielsweise eine exakte Position des verschiebbaren Elements, beispielsweise des verschieblichen Stopfens und/oder eines Kolbens, relativ zu dem Behälter erfasst werden. Somit kann eine übliche Steuerung eines Vortriebs beispielsweise durch Regelung des Vortriebs ersetzt werden, da beispielsweise die erfasste relative Positionierung (Ist-Wert) mit mindestens einem SollWert verglichen werden kann. Im Gegensatz zu herkömmlichen Insulinpumpen, bei welchen eine Steuerung eines Vortriebs erfolgt, kann nunmehr beispielsweise eine Regelung des Vortriebs erfolgen. Auf diese Weise kann die Dosierung exakter gestaltet werden, da beispielsweise das fluide Medium unter konstantem Druck stehen kann und die Verschiebung beispielsweise als exaktes Maß für die abgegebene Menge an fluidem Medium dienen kann.

Weiterhin kann, beispielsweise innerhalb eines Gehäuses der Medikationsvorrichtung, eine relative Verschiebung des Behälters gemessen werden, was technisch einfacher realisierbar ist als die Messung einer Verschiebung des verschiebbaren Elements in dem Behälter, beispielsweise als die Messung der Verschiebung eines Stopfens in dem Behälter.

Weiterhin kann die Beeinflussung der Abgabe des fluiden Mediums durch das Stellglied auf einfache und zuverlässige Weise realisiert werden. Beispielsweise kann, wie oben beschrieben, eine Ventilsteuerung die Abgabe des fluiden Mediums, beispielsweise die Dosierung, übernehmen. So kann beispielsweise ein einfaches Auf-zu-Ventil verwendet werden, über welches eine Abgabemenge bestimmt werden kann. Allgemein kann beispielsweise das Messelement eine Regelfunktion übernehmen und/oder Bestandteil eines Regelkreises sein und/oder mindestens einen Regelparameter abbilden. Zu einer Ventilsteuerung wird erheblich weniger Energie benötigt als beispielsweise für eine Steuerung eines Vortriebs. Hierdurch lassen sich längere Standzeiten der Medikationsvorrichtung und/oder kleinere elektrische Energiespeicher wie beispielsweise kleinere Batterien realisieren.

Weiterhin kann die Medikationsvorrichtung, wie oben ausgeführt, eigensicher ausgestaltet werden. So kann das optionale mindestens eine Ventil beispielsweise derart konstruiert werden, dass dieses von einer geschlossenen Stellung in eine andere geschlossene Stellung übergeht, sodass lediglich bei einer aktiven Bewegung zwischen den beiden geschlossenen Stellungen, beispielsweise zwischen den Stopp-Positionen, ein minimaler Durchfluss auftreten kann. Bei einem mechanischen und/oder elektrischen Versagen von Bauteilen kann somit ein unkontrollierter Durchfluss ausgeschlossen werden.

Ein weiterer Vorteil besteht darin, dass grundsätzlich auf ein Entlüften (Priming) verzichtet werden kann. So kann beispielsweise gemäß dem oben beschriebenen Vorschlag ein Transport- und Transferbehältnis verwendet werden, in welchem das fluide Medium blasenfrei aufgenommen ist, beispielsweise durch Beaufschlagung desselben mit einem Überdruck. Durch Verwendung von blasenfreien Transport- und Transferbehältnissen besteht grundsätzlich keine Gefahr von unbestimmten Luftblasen. Weiterhin ist das Volumen der Fluidverbindung und optional einer Injektionsnadel in der Regel bekannt. So kann eine Injektionsnadel weiterer Bestandteil der Medikationsvorrichtung sein. Dieses Volumen der Fluidverbindung, beispielsweise des Injektionsschlauchs, und optional der Injektionsnadel kann weiterhin optimiert werden und beispielsweise bei der ersten Dosis, die mit der Medikationsvorrichtung abgegeben wird, berücksichtigt werden. So kann ein aktives Entlüften der Medikationsvorrichtung durch den Benutzer entfallen. Ein geringes injiziertes Luftvolumen ist insbesondere bei einer subkutanen Injektion jedoch grundsätzlich problemlos. Ein Vorbereitungsschritt kann grundsätzlich ersatzlos entfallen, und das Risiko einer potentiellen Fehlfunktion, beispielsweise durch Luftinjektion oder eine falsche Insulinmenge, kann minimiert oder ausgeschlossen werden.

Weiterhin lässt sich bei der vorgeschlagenen Medikationsvorrichtung insbesondere auch ein Nachfüllen und/oder Austauschen des fluiden Mediums, beispielsweise des Transport-und Transferbehältnisses, einfach ausgestalten. So kann beispielsweise das Transport- und Transferbehältnis und/oder der Behälter desselben einfach gegen ein volles Transport- und Transferbehältnis und/oder einen vollen Behälter ausgetauscht werden. Dieser Austausch kann derart erfolgen, dass kein Lufteintrag oder lediglich ein vernachlässigbarer Lufteintrag in die Medikationsvorrichtung erfolgt. Auch hierbei kann grundsätzlich ein Priming entfallen.

Ein weiterer Vorteil besteht darin, dass das Injektionssystem in definierte Module, welche jeweils eine eigene Baugruppe bilden können, unterteilt werden können. Als erstes Modul kann beispielsweise das Transport- und Transferbehältnis dienen, welches beispielsweise vorgefüllt sein kann oder welches von einem Benutzer gefüllt sein kann. Bei einem vorgefüllten Transport- und Transferbehältnis kann beispielsweise eine Energieeinheit vorgesehen sein, welche für eine Beaufschlagung mit Überdruck sorgt. Das Transport- und Transferbehältnis kann beispielsweise einen Bedarf für eine vorgegebene Zeitspanne bereitstellen, beispielsweise einen Tagesbedarf.

Während das Transport- und Transferbehältnis vorzugsweise ganz oder teilweise als Einwegteil ausgestaltet werden kann, kann das oben beschriebene optionale Steuermodul als wiederverwendbare Steuerungseinheit ausgestaltet sein, beispielsweise für eine Steuerung des Stellglieds und insbesondere für eine Ventilstellung und optional für ein Datenmanagement. So kann beispielsweise das Steuermodul eingerichtet sein, um ein Datenmanagement für einen Zeitraum von 6-12 Monaten zu gewährleisten. Beispielsweise kann das Steuermodul mindestens eine Datenverarbeitungsvorrichtung und/oder mindestens einen Datenspeicher umfassen. Weiterhin kann das Steuermodul ein oder mehrere Bedienelemente umfassen, beispielsweise eine oder mehrere Tasten und/oder eine oder mehrere andere Arten von Bedienelementen. Weiterhin können ein oder mehrere andere Arten von Benutzerschnittstellen vorgegeben sein, beispielsweise Datenschnittstellen und/oder ein oder mehrere Elemente für eine visuelle und/oder akustische und/oder haptische Ausgabe von Daten und/oder Informationen.

Die Medikationsvorrichtung kann insbesondere derart ausgestaltet sein, dass das Steuermodul für eine Vielzahl von Verwendungszyklen vorgesehen ist, mit einer Vielzahl von Transport- und Transferbehältnissen, welche nacheinander, beispielsweise jeweils bis zu einer Entleerung, direkt oder indirekt mit dem Steuermodul verbunden werden können. Auf diese Weise kann beispielsweise jeweils eine Einmalinjektionseinheit mit einem Infusionsset und einer eigensicheren Ventilvorrichtung realisiert werden, welche zum Anschluss an die Steuerungseinheit und zur Verwendung von mehreren vorgefüllten Transport- und Transferbehältnissen, beispielsweise für eine Zeitdauer von 5-10 Tagen, ausgestaltet werden kann. Weiterhin besteht auch die Möglichkeit, unter Verwendung der vorgeschlagenen Baugruppen eine so genannte Patch-Pumpe aufzubauen, welche beispielsweise direkt oder indirekt auf eine Hautoberfläche des Benutzers aufgebracht werden kann.

Insgesamt lassen sich erfindungsgemäß die Baugröße, das Gewicht und die Mechanik der Medikationsvorrichtung gegenüber bekannten Medikationsvorrichtungen stark reduzieren. So ermöglicht insbesondere ein modularer Aufbau, beispielsweise der oben beschriebene Aufbau mit den vorgeschlagenen Baugruppen, den Aufbau von Medikationsvorrichtungen und insbesondere Medikationspumpen mit einem geringeren Mechanikanteil, einem geringeren Energiebedarf und einem geringeren Gewicht im Vergleich zu herkömmlichen Medikationsvorrichtungen. Da auch ein Austausch und/oder ein Nachfüllen des Transport-und Transferbehältnisses erheblich vorteilhafter realisiert werden kann, sind auch kleinere Transport- und Transferbehältnisse, beispielsweise mit einem Tagesbedarf von maximal 1 ml, vorteilhaft, die wiederum eine Baugröße und ein Gewicht reduzieren können. Durch die Verwendung eines vorgefüllten Transport- und Transferbehältnisses kann eine Handhabung sehr einfach und sicher ausgestaltet werden, und es kann zusätzlich in der Regel eine blasenfreie Abgabe des fluiden Mediums, beispielsweise eine blasenfreie Insulinabgabe, gewährleistet werden.

Die Befüllung der Medikationsvorrichtung und/oder des Transport- und Transferbehältnisses kann herstellerseitig erfolgen. Alternativ oder zusätzlich besteht auch die Möglichkeit, die Medikationsvorrichtung und/oder das Transport- und Transferbehältnis durch einen Benutzer manuell durchführen zu lassen. Insgesamt lässt sich beispielsweise auf diese Weise eine sehr nutzerfreundliche Insulinpumpe realisieren, bei welcher günstigere Einweg-Module vorgesehen werden können. Insgesamt können sich steriltechnische Vorteile und eine gute Produktstabilität durch die Verwendung von einfachen und bekannten Materialien ergeben.

Weitere Vorteile ergeben sich aus der Erfassung der relativen Positionierung des verschiebbaren Elements zu dem Behälter mittels des Messelements. So kann insbesondere eine Messung eines abgegebenen Flüssigkeitsvolumens aus einem unter Druck stehenden Behälter mittels einer Messung einer Längsverschiebung es Behälters erfolgen. Dieses direkte Messverfahren weist in der Regel keine Hysterese oder lediglich eine geringfügige Hysterese auf und ist somit präziser als derzeit eingesetzte Verfahren, bei denen in der Regel Motorumdrehungen als ein indirektes Maß für eine abgegebene Menge des fluiden Mediums verwendet werden. Die Messung einer relativen Positionierung, beispielsweise eine Längenmessung, setzt kein Vorhandensein eines fluiden Mediums unter Druck voraus. Beispielsweise kann eine herkömmliche Carpule in Verbindung mit einer Mikropumpe verwendet werden, wobei beispielsweise ein Kolben in die Carpule gezogen wird und als Maß einer abgegebenen Flüssigkeitsmenge dienen kann. Beispielsweise können zu diesem Zweck bekannte, vom Anwender zu füllende Behältnisse, welche bereits heute im Einsatz sind, verwendet werden. Das mindestens eine Messelement kann also allgemein insbesondere für eine Längenmessung ausgestaltet sein. Mittels dieser Längenmessung kann beispielsweise eine Regelung der Stellglieds, beispielsweise eines Ventils und/oder einer Pumpe, erfolgen. Die Längenmessung kann beispielsweise derart erfolgen, dass das Transport- und Transferbehältnis eine Spritzenform aufweist und die Längenmessung in der Röhre des Behälters oder durch eine Behälterwand hindurch stattfindet. Alternativ oder zusätzlich kann das Transport- und Transferbehältnis jedoch auch lediglich einen perforierbaren Stopfen aufweisen, und eine Längenmessung kann beispielsweise von außen stattfinden.

Wird ein Transport- und Transferbehältnis verwendet, bei welchem das fluide Medium flüssig ist und unter einem Überdruck steht, so kann, wie oben beschrieben, eine Gasblasenbildung zumindest weitgehend vermieden werden. Insbesondere kann weiterhin eine Pumpe entfallen und/oder durch ein Ventil ersetzt werden.

Ein weiterer Vorteil ergibt sich, wie oben ausgeführt, aus der Möglichkeit, das Stellglied ganz oder teilweise als eigensicheres Ventil auszugestalten. Bei einem eigensicheren Ventil kann beispielswese ein flexibler und/oder elastischer Abgabeschlauch der Fluidverbindung gequetscht werden, insbesondere zur Steuerung einer Flüssigkeitsabgabemenge. Eine eigensichere Ausgestaltung kann gewährleisten, dass beispielsweise eine Steuerung immer von einer geschlossenen zu einer geschlossenen Position fährt und lediglich während der Bewegung Flüssigkeit abgibt. Auch hier ist es, wie oben ausgeführt, vorteilhaft, elastische Schläuche mit großer Wandstärke und kleinem Innendurchmesser einzusetzen, da bei der Konstruktion in der Regel keine stationäre, nicht-dynamische Position auftritt, bei welcher Flüssigkeit ungewollt austreten kann.

Weitere Vorteile ergeben sich aus dem oben beschriebenen optionalen Konzept einer Flüssigkeitsabgabe. Wie oben beschrieben, kann das Stellglied beispielsweise mindestens zwei Aktuatoren und einen dehnbaren Bereich eines Schlauchabschnitts umfassen. Auf diese Weise lässt sich ein besonders einfaches Prinzip einer Flüssigkeitsabgabe realisieren. Wie oben ausgeführt, kann beispielsweise bei einem geschlossenen auslassseitigen Aktuator, beispielsweise bei einem geschlossenen Ausgabeventil, Flüssigkeit über einen einlassseitigen Aktuator, beispielsweise ein geöffnetes Einlassventil, unter Druck in den dehnbaren Bereich, beispielsweise einen flexiblen und/oder elastischen Schlauchabschnitt, eingebracht werden. Auf diese Weise kann sich der dehnbare Bereich, beispielsweise der Schlauch, ausdehnen. Bei geschlossenem einlassseitigem Aktuator und geöffnetem auslassseitigem Aktuator kann sich der dehnbare Bereich dann entspannen, wobei ein kleines Flüssigkeitsaliquot abgegeben wird. Der dehnbare Bereich wird dabei in der Regel nicht vollständig geleert, sondern die abgegeben Menge entspricht in der Regel der Dehnung des dehnbaren Bereichs des Schlauchabschnitts. Hierdurch kann eine Abgabe kleinster Flüssigkeitsmengen realisierbar sein. Vorteilhaft ist es dabei, elastische Schläuche mit großer Wandstärke und kleinem Innendurchmesser einzusetzen. Dieses Prinzip kann beispielsweise einer linearen Peristaltikpumpe entsprechen, welche beispielsweise zwei Elemente einsetzt, die eine bestehende Druckdifferenz ausnutzen kann.

Zusammenfassend werden im Rahmen der vorliegenden Erfindung folgende Ausgestaltungen als besonders vorteilhaft angesehen:
Ausführungsform 1: Medikationsvorrichtung zur dosierten Abgabe eines fluiden Mediums, insbesondere eines fluiden Therapeutikums und/oder Diagnostikums, insbesondere Insulinpumpe, umfassend:
a) mindestens ein Transport- und Transferbehältnis, wobei das Transport- und Transferbehältnis mindestens einen Behälter zur Aufnahme des fluiden Mediums aufweist, wobei der Behälter mindestens ein verschiebbares Element aufweist, wobei das verschiebbare Element relativ zu dem Behälter beweglich gelagert ist und wobei eine relative Positionierung des verschiebbaren Elements zu dem Behälter einen zur Aufnahme des fluiden Mediums zur Verfügung stehenden Innenraum des Behälters bestimmt,
b) mindestens ein Messelement, wobei das Messelement eingerichtet ist, um die relative Positionierung des verschiebbaren Elements zu dem Behälter zu erfassen, und
c) mindestens ein Stellglied zur Beeinflussung einer Abgabe des fluiden Mediums über mindestens eine Fluidverbindung zu dem Innenraum,
wobei das Messelement und das Stellglied derart zusammenwirken, dass die Abgabe des fluiden Mediums durch die von dem Messelement erfasste relative Positionierung beeinflusst wird.

Ausführungsform 2: Medikationsvorrichtung nach der vorhergehenden Ausführungsform, wobei das Messelement eingerichtet ist, um mindestens ein Messsignal entsprechend der relativen Positionierung zu erzeugen, wobei die Medikationsvorrichtung mindestens eine Steuerung aufweist, wobei die Steuerung eingerichtet ist, um das Stellglied mittels des Messsignals zu steuern und/oder zu regeln.

Ausführungsform 3: Medikationsvorrichtung nach einer der vorhergehenden Ausführungsformen, wobei die Medikationsvorrichtung eingerichtet ist, um eine abgegebene Absolutmenge und/oder eine Abgaberate des fluiden Mediums durch die von dem Messelement erfasste relative Positionierung zu beeinflussen, insbesondere einzustellen, zu steuern oder zu regeln.

Ausführungsform 4: Medikationsvorrichtung nach einer der vorhergehenden Ausführungsformen, wobei das Messelement eingerichtet ist, um die relative Positionierung des verschiebbaren Elements zu dem Behälter direkt zu erfassen.

Ausführungsform 5: Medikationsvorrichtung nach einer der vorhergehenden Ausführungsformen, wobei das verschiebbare Element einen relativ zu dem Behälter verschiebbaren Stopfen umfasst.

Ausführungsform 6: Medikationsvorrichtung nach der vorhergehenden Ausführungsform, wobei das Messelement eingerichtet ist, um eine Verschiebung des Stopfens in dem Behälter, insbesondere eine lineare Verschiebung, zu erfassen.

Ausführungsform 7: Medikationsvorrichtung nach einer der vorhergehenden Ausführungsformen, wobei das Messelement ausgewählt ist aus der Gruppe bestehend aus einem mechanischen Messelement, einem magnetischen Messelement, einem elektronischen Messelement, einem induktiven Messelement, einem kapazitiven Messelement, einem resistiven Messelement, einem optischen Messelement und einem Ultraschall-Messelement.

Ausführungsform 8: Medikationsvorrichtung nach einer der vorhergehenden Ausführungsformen, wobei das Messelement eingerichtet ist, um mindestens eine Position mindestens einer Grenzfläche zwischen dem verschiebbaren Element und dem fluiden Medium zu erfassen.

Ausführungsform 9: Medikationsvorrichtung nach einer der vorhergehenden Ausführungsformen, wobei das Messelement eingerichtet ist, um die relative Positionierung mit einer Genauigkeit von mindestens 0,05 mm, insbesondere von mindestens 0,01 mm zu erfassen.

Ausführungsform 10: Medikationsvorrichtung nach einer der vorhergehenden Ausführungsformen, wobei die Fluidverbindung das verschiebbare Element, insbesondere den Stopfen, durchdringt.

Ausführungsform 11: Medikationsvorrichtung nach der vorhergehenden Ausführungsform, wobei das verschiebbare Element einen perforierbaren Stopfen umfasst, wobei die Fluidverbindung mindestens eine Kanüle umfasst, welche den perforierbaren Stopfen durchdringt.

Ausführungsform 12 (erfindungsgemäß): Medikationsvorrichtung, wobei die Medikationsvorrichtung mindestens ein Gehäuse aufweist, wobei der Behälter beweglich in dem Gehäuse aufgenommen ist und wobei das verschiebbare Element relativ zu dem Gehäuse fixiert ist, wobei das Messelement eingerichtet ist, um eine relative Positionierung des Behälters in dem Gehäuse zu erfassen.

Ausführungsform 13: Medikationsvorrichtung nach einer der vorhergehenden Ausführungsformen, wobei die Medikationsvorrichtung eingerichtet ist, um das verschiebbare Element und/oder den Behälter derart mit einer Kraft zu beaufschlagen, dass ein Überdruck auf das fluide Medium ausgeübt wird und/oder dass das fluide Medium unter einem erhöhten Druck steht.

Ausführungsform 14: Medikationsvorrichtung nach der vorhergehenden Ausführungsform, wobei die Medikationsvorrichtung eingerichtet ist, um das verschiebbare Element gegen ein Widerlager zu pressen, so dass das verschiebbare Element in den Innenraum hinein gedrückt wird.

Ausführungsform 15: Medikationsvorrichtung nach der vorhergehenden Ausführungsform, wobei die Fluidverbindung durch das Widerlager hindurch erfolgt.

Ausführungsform 16: Medikationsvorrichtung nach einer der vorhergehenden Ausführungsformen, wobei die Medikationsvorrichtung mindestens eine Energieeinheit aufweist, wobei die Energieeinheit eingerichtet ist, um das fluide Medium in dem Behälter mit einem Überdruck zu beaufschlagen.

Ausführungsform 17: Medikationsvorrichtung nach der vorhergehenden Ausführungsform, wobei die Energieeinheit mindestens einen Energiespeicher aufweist, insbesondere mindestens einen mechanischen Energiespeicher und besonders bevorzugt mindestens ein Federelement.

Ausführungsform 18: Medikationsvorrichtung nach einer der beiden vorhergehenden Ausführungsformen, wobei die Energieeinheit eingerichtet ist, um den Druck für eine Zeitdauer von mindestens einem Monat, vorzugsweise für eine Zeitdauer von mindestens sechs Monaten und besonders bevorzugt von mindestens einem Jahr aufrechtzuerhalten.

Ausführungsform 19: Medikationsvorrichtung nach einer der drei vorhergehenden Ausführungsformen, wobei die Energieeinheit Bestandteil des Transport- und Transferbehältnisses ist.

Ausführungsform 20: Medikationsvorrichtung nach der vorhergehenden Ausführungsform, wobei das Transport- und Transferbehältnis in einem druckbeaufschlagten Zustand unabhängig von weiteren Komponenten der Medikationsvorrichtung gehandhabt werden kann.

Ausführungsform 21: Medikationsvorrichtung nach einer der vorhergehenden Ausführungsformen, wobei das Stellglied mindestens ein Ventil aufweist, wobei das Ventil eingerichtet ist, um einen Durchfluss des fluiden Mediums durch die Fluidverbindung zu beeinflussen, insbesondere wahlweise zu unterbrechen oder freizugeben.

Ausführungsform 22: Medikationsvorrichtung nach der vorhergehenden Ausführungsform, wobei die Fluidverbindung mindestens eine Schlauchverbindung aufweist, insbesondere eine flexible Schlauchverbindung, wobei das Ventil mindestens ein Ventilglied aufweist, wobei das Ventilglied eingerichtet ist, um die Schlauchverbindung zu quetschen.

Ausführungsform 23: Medikationsvorrichtung nach der vorhergehenden Ausführungsform, wobei die Schlauchverbindung zumindest partiell eine Wandstärke aufweist, welche einen Innendurchmesser der Schlauchverbindung überschreitet.

Ausführungsform 24: Medikationsvorrichtung nach einer der drei vorhergehenden Ausführungsformen, wobei das Ventil mindestens zwei geschlossene Ventilstellungen aufweist, wobei ein Durchfluss des fluiden Mediums unterbrochen ist, wenn das Ventil in einer der geschlossenen Ventilstellungen ist.

Ausführungsform 25: Medikationsvorrichtung nach der vorhergehenden Ausführungsform, wobei die geschlossenen Ventilstellungen Quetschpositionen sind, wobei in den Quetschpositionen die Fluidverbindung, insbesondere eine Schlauchverbindung der Fluidverbindung, gequetscht wird.

Ausführungsform 26: Medikationsvorrichtung nach einer der beiden vorhergehenden Ausführungsformen, wobei die geschlossenen Ventilstellungen Endstellungen einer Bewegung mindestens eines Ventilglieds des Ventils umfassen, wobei sich das Ventilglied zwischen den Ventilstellungen bewegen kann.

Ausführungsform 27: Medikationsvorrichtung nach der vorhergehenden Ausführungsform, wobei das Ventilglied ein um eine Achse drehbar gelagertes Ventilglied umfasst, welches über mindestens zwei Druckelemente, insbesondere über zwei Quetschelemente, die Fluidverbindung unterbrechen kann.

Ausführungsform 28: Medikationsvorrichtung nach einer der sieben vorhergehenden Ausführungsformen, wobei sich das Ventil in einer Ruhestellung, in welcher keine Kraft auf ein Ventilglied des Ventils ausgeübt wird, in einer geschlossenen Ventilstellung befindet, in welcher der Durchfluss des fluiden Mediums durch die Fluidverbindung unterbrochen ist.

Ausführungsform 29: Medikationsvorrichtung nach der vorhergehenden Ausführungsform, wobei die Fluidverbindung mindestens eine Schlauchverbindung aufweist, wobei in der Ruhestellung das Ventilglied die Schlauchverbindung in mindestens zwei Quetschpositionen quetscht und dadurch die Fluidverbindung in den mindestens zwei Quetschpositionen unterbricht.

Ausführungsform 30: Medikationsvorrichtung nach einer der neun vorhergehenden Ausführungsformen, wobei das Ventil mindestens ein Ventilglied aufweist, wobei das Ventilglied in mindestens zwei statische Ventilpositionen eingestellt werden kann, wobei in allen statischen Ventilpositionen ein Durchfluss des fluiden Mediums durch die Fluidverbindung unterbrochen ist.

Ausführungsform 31: Medikationsvorrichtung nach einer der zehn vorhergehenden Ausführungsformen, wobei das Ventil mindestens zwei Ventilstellungen aufweist, insbesondere mindestens zwei Ventilstellungen mindestens eines Ventilglieds des Ventils, wobei die Ventilstellungen jeweils derart ausgestaltet sind, dass der Durchfluss des fluiden Mediums jeweils an mindestens einer anderen Stelle der Fluidverbindung unterbrochen ist.

Ausführungsform 32: Medikationsvorrichtung nach einer der vorhergehenden Ausführungsformen, wobei das Stellglied mindestens eine Pumpe aufweist.

Ausführungsform 33: Medikationsvorrichtung nach der vorhergehenden Ausführungsform, wobei die Pumpe eine Peristaltikpumpe aufweist.

Ausführungsform 34: Medikationsvorrichtung nach einer der vorhergehenden Ausführungsformen, wobei das Stellglied mindestens zwei Aktuatoren umfasst, wobei die Fluidverbindung mindestens einen Schlauchabschnitt umfasst, wobei die Aktuatoren eingerichtet sind, um den Schlauchabschnitt in mindestens zwei verschiedenen Quetschbereichen zu quetschen.

Ausführungsform 35: Medikationsvorrichtung nach der vorhergehenden Ausführungsform, wobei zwischen den Aktuatoren ein dehnbarer Bereich des Schlauchabschnitts angeordnet ist.

Ausführungsform 36: Medikationsvorrichtung nach der vorhergehenden Ausführungsform, wobei die Aktuatoren einen einlassseitigen Aktuator und einen auslassseitigen Aktuator umfassen, wobei die Medikationsvorrichtung eingerichtet ist, um bei geöffnetem einlassseitigem Aktuator und geschlossenem auslassseitigem Aktuator fluides Medium unter Druck in den dehnbaren Bereich zwischen den Aktuatoren einzulassen, wobei der dehnbare Bereich gedehnt wird, wobei die Medikationsvorrichtung weiterhin eingerichtet ist, um anschließend den einlassseitigen Aktuator zu schließen und den auslassseitigen Aktuator zu öffnen, wobei der dehnbare Bereich entspannt wird und ein Teil des fluiden Mediums aus dem dehnbaren Bereich ausströmt.

Ausführungsform 37: Medikationsvorrichtung nach einer der vorhergehenden Ausführungsformen, wobei die Medikationsvorrichtung modular aufgebaut ist, wobei die Medikationsvorrichtung mindestens ein Steuermodul mit einer Steuerung umfasst, wobei das Transport- und Transferbehältnis mindestens ein weiteres Modul der Medikationsvorrichtung bildet, wobei das Transport- und Transferbehältnis austauschbar ausgestaltet ist.

Ausführungsform 38: Medikationsvorrichtung nach der vorhergehenden Ausführungsform, wobei die Medikationsvorrichtung weiterhin mindestens ein Basismodul aufweist mit mindestens einer Basisplatte, wobei das Steuermodul und das Transport- und Transferbehältnis direkt oder indirekt mit der Basisplatte verbindbar sind.

Ausführungsform 39: Medikationsvorrichtung nach einer der beiden vorhergehenden Ausführungsformen, wobei die Medikationsvorrichtung weiterhin mindestens ein Fluidikmodul umfasst, wobei das Fluidikmodul zumindest einen Teil der Fluidverbindung umfasst.

Ausführungsform 40: Medikationsvorrichtung nach der vorhergehenden Ausführungsform, wobei das Fluidikmodul als Einwegteil ausgestaltet ist.

Ausführungsform 41: Medikationsvorrichtung nach einer der vier vorhergehenden Ausführungsformen, wobei das Transport- und Transferbehältnis derart ausgestaltet ist, dass die Fluidverbindung bei einer Verbindung der Module herstellbar ist, insbesondere durch eine Perforation des verschiebbaren Elements.

Ausführungsform 42: Verfahren zur dosierten Abgabe eines fluiden Mediums, insbesondere eines fluiden Therapeutikums und/oder Diagnostikums, insbesondere unter Verwendung einer Medikationsvorrichtung nach einer der vorhergehenden Ausführungsformen,
- wobei mindestens ein Transport- und Transferbehältnis verwendet wird, wobei das Transport- und Transferbehältnis mindestens einen Behälter zur Aufnahme des fluiden Mediums aufweist, wobei der Behälter mindestens ein verschiebbares Element aufweist, beispielsweise durch mindestens ein verschiebbares Element in Form mindestens eines Verschlusselements verschlossen wird, wobei das verschiebbare Element relativ zu dem Behälter beweglich gelagert wird und wobei eine relative Positionierung des verschiebbaren Elements zu dem Behälter einen zur Aufnahme des fluiden Mediums zur Verfügung stehenden Innenraum des Behälters bestimmt,
- wobei mittels mindestens eines Messelements die relative Positionierung des verschiebbaren Elements zu dem Behälter erfasst wird,
- wobei weiterhin mindestens ein Stellglied zur Beeinflussung einer Abgabe des fluiden Mediums über mindestens eine Fluidverbindung zu dem Innenraum verwendet wird,
- wobei das Messelement und das Stellglied derart zusammenwirken, dass die Abgabe des fluiden Mediums durch die von dem Messelement erfasste relative Positionierung beeinflusst wird.

Ausführungsform 43: Verwendung einer Medikationsvorrichtung nach einer der vorhergehenden, eine Medikationsvorrichtung betreffenden Ausführungsformen zur Dosierung eines Therapeutikums und/oder Diagnostikums, insbesondere Insulin.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

### Im Einzelnen zeigen:

- Figur 1A: ein Ausführungsbeispiel einer erfindungsgemäßen Medikationsvorrichtung;
- Figur 1B: eine vergrößerte Darstellung eines Transport- und Transferbehältnisses zum Einsatz in einer Medikationsvorrichtung beispielsweise gemäß Figur 1A;
- Figur 2: eine Detaildarstellung eines alternativen Stellglieds zum Einsatz in einer Medikationsvorrichtung;
- Figuren 3A-3F: verschiedene Darstellungen eines weiteren Ausführungsbeispiels eines Stellglieds zum Einsatz in einer erfindungsgemäßen Medikationsvorrichtung;
- Figuren 4A und 4B: verschiedene Ansichten einer modular zusammengesetzten Medikationsvorrichtung; und
- Figur 5: ein alternatives Ausführungsbeispiel einer Medikationsvorrichtung mit einem Stellglied in Form einer Pumpe.

### Ausführungsbeispiele

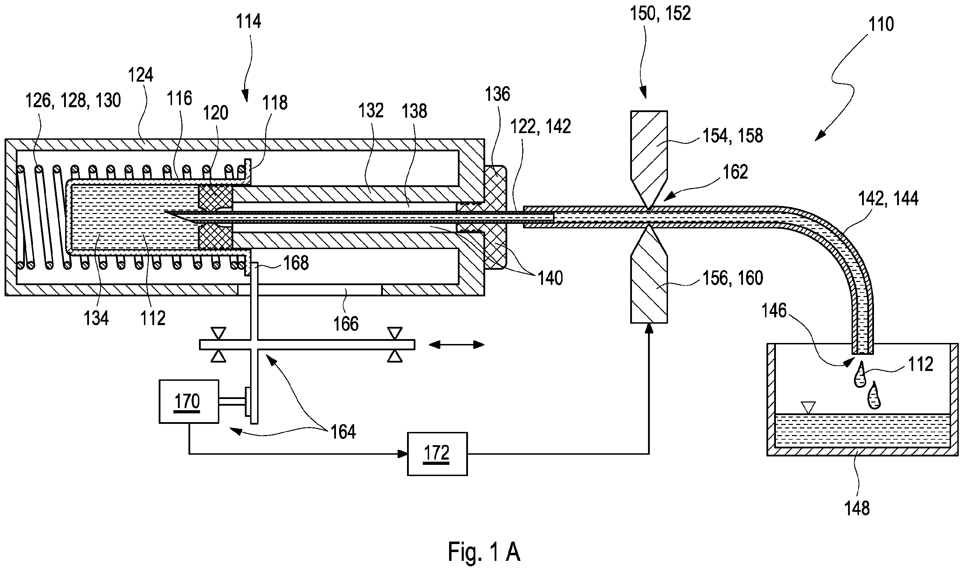

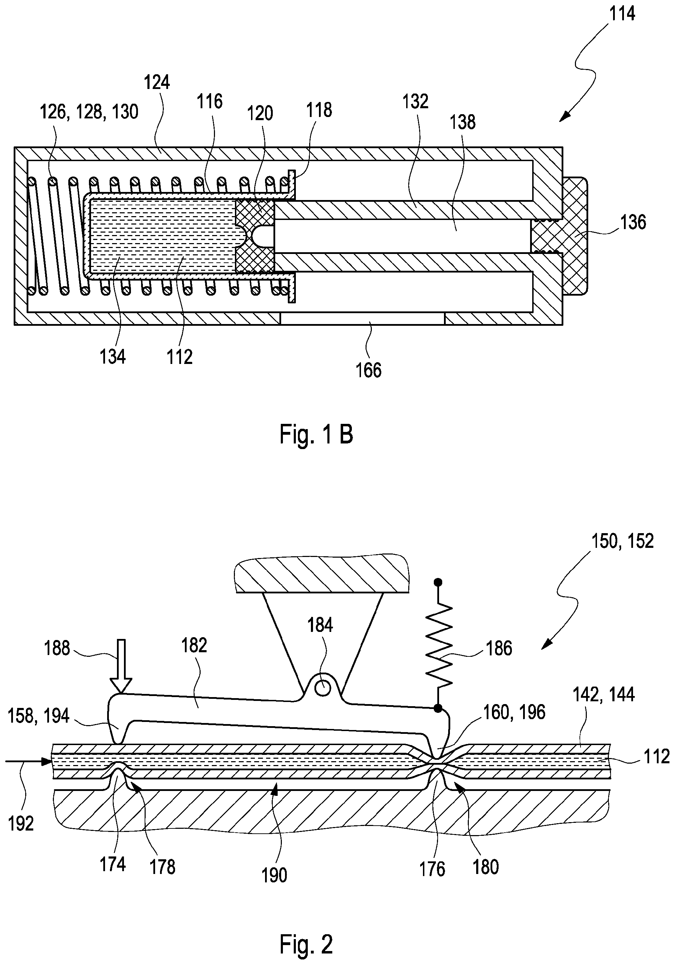

In Figur 1A ist ein erstes Ausführungsbeispiel einer Medikationsvorrichtung 110 zur dosierten Abgabe eines fluiden Mediums 112 in einer Schnittdarstellung gezeigt. Die Medikationsvorrichtung 110 umfasst ein Transport- und Transferbehältnis 114, welches exemplarisch in Figur 1B in einem Lieferzustand dargestellt ist, ebenfalls in einer Schnittdarstellung. Auf beide Figuren wird im Folgenden gemeinsam Bezug genommen.

Das Transport- und Transferbehältnis umfasst in dem dargestellten Ausführungsbeispiel exemplarisch einen Behälter 116, beispielsweise aus einem Glasmaterial oder einem Kunststoffmaterial. Der Behälter 116 kann beispielsweise, wie in den Figuren 1A und 1B dargestellt, als einseitig geschlossene Röhre ausgestaltet sein. Der Behälter 116 kann beispielsweise einen vorspringenden umlaufenden Rand 118 aufweisen und ist durch ein in dem Behälter 116 beweglich gelagertes verschiebbares Element 120 verschlossen. Beispielsweise kann es sich bei diesem verschiebbaren Element 120 um einen verschiebbaren Stopfen handeln, insbesondere einen perforierbaren Stopfen. Während in der Darstellung gemäß Figur 1B, welche das Transport- und Transferbehältnis 114 im Lieferzustand zeigt, der Stopfen noch nicht perforiert ist, zeigt Figur 1A einen Zustand, in welchem das Transport- und Transferbehältnis 114 in der Medikationsvorrichtung 110 aufgenommen ist und in welchem das verschiebbare Element 120 durch ein Perforationselement 122, beispielsweise eine Hohlnadel und/oder eine Kanüle, perforiert ist.

Der Behälter 116 ist mit dem fluiden Medium 112 befüllt. Der Behälter 116 ist in einem Behältergehäuse 124 aufgenommen, welches beispielsweise ganz oder teilweise transparent ausgestaltet sein kann, wie auch der Behälter 116 selbst, sodass ein Benutzer beispielsweise einen Füllstand des Behälters 116 optisch überprüfen kann.

Weiterhin umfasst optional das Transport- und Transferbehältnis 114 in dem dargestellten Ausführungsbeispiel eine Energieeinheit 126, welche eingerichtet ist, um das fluide Medium 112 in dem Behälter 116 mit einem Druck zu beaufschlagen. Beispielsweise kann diese Druckbeaufschlagung unmittelbar in einen Herstellungsprozess integriert werden, sodass das Transport- und Transferbehältnis 114 bereits in einem Zustand angeliefert werden kann, beispielsweise verpackt, in welchem das fluide Medium 112 mit einem Überdruck beaufschlagt ist. Die Energieeinheit 126 umfasst in dem dargestellten Ausführungsbeispiel einen Energiespeicher 128, beispielsweise einen mechanischen Energiespeicher in Form eines Federelements 130. Dieses Federelement 130 kann beispielsweise, wie in den Figuren 1A und 1B dargestellt, das verschiebbare Element 120 gegen ein Widerlager 132 pressen, sodass das verschiebbare Element 120 in einen Innenraum 134 des Behälters 116 hineingedrückt wird und das fluide Medium 112 somit mit dem Überdruck beaufschlagt. Das Widerlager 132 kann beispielsweise in diesem oder auch in anderen Ausführungsbeispielen Bestandteil des Behältergehäuses 124 und/oder anderer Teile der Medikationsvorrichtung 110 sein. Beispielsweise kann das Widerlager 132 röhrenförmig ausgestaltet sein. Dabei zeigt Figur 1B eine Darstellung, in welcher das röhrenförmige Widerlager durch eine Abdichtung 136 verschlossen ist. Diese Abdichtung 136, welche beispielsweise wiederum als Stopfen ausgestaltet sein kann, kann beispielsweise als Sterilschutz dienen und kann vor einem Einsetzen des Transport- und Transbehältnisses 114 in die Medikationsvorrichtung 110 entfernt werden, sodass eine Aussparung oder ein Hohlraum 138 des Widerlagers 132 freigegeben wird. Durch diesen Hohlraum 138 kann wiederum, wie in Figur 1A dargestellt, das Perforationselement 122 eingeführt werden, beispielsweise mittels einer in Figur 1A ebenfalls optional dargestellten Führung 140, die beispielsweise eine Zentrierung des Perforationselement 122 bewirkt. Bei diesem Einführen perforiert das Perforationselement 122 das verschiebbare Element 120 und stellt eine Fluidverbindung zum Innenraum 134 her.

Während eines Transports und einer Lagerung kann das fluide Medium 112 in dem dargestellten Ausführungsbeispiel somit mit einem Überdruck beaufschlagt sein. Dieser Überdruck kann beispielsweise bis zu einem Verbrauch aufrechterhalten werden. Insbesondere kann die Aufnahme des fluiden Mediums 112 in dem Behälter 116 blasenfrei erfolgen. Beispielsweise kann das fluide Medium ein Therapeutikum und/oder ein Diagnostikum umfassen, insbesondere Insulin, welches vorzugsweise blasenfrei in dem Behälter 116 aufgenommen sein kann.

Sobald die Perforation des verschiebbaren Elements 120 stattgefunden hat, wie in Figur 1A dargestellt, kann das fluide Medium über eine Fluidverbindung 142 abgegeben werden. Diese Fluidverbindung 142 kann, beispielsweise neben dem optionalen Perforationselement 122, mindestens eine Schlauchverbindung 144 umfassen. Diese Schlauchverbindung 144 kann beispielsweise das fluide Medium 112 hin zu einer Abgabestelle 146 führen, an welcher das fluide Medium 112 dosiert bereitgestellt wird. Im dargestellten Ausführungsbeispiel in Figur 1A erfolgt die Bereitstellung exemplarisch in ein Gefäß 148. Auch eine andere Art der Bereitstellung ist jedoch grundsätzlich möglich, beispielsweise eine Bereitstellung, bei welcher die Schlauchverbindung 144 direkt oder über ein oder mehrere Zwischenelemente an ein weiteres Gerät angeschlossen ist.

Die Medikationsvorrichtung umfasst weiterhin ein Stellglied 150 zur Beeinflussung einer Abgabe des fluiden Mediums über die Fluidverbindung 142. Dieses Stellglied kann beispielsweise, wie in Figur 1A exemplarisch dargestellt, ein Ventil 152 umfassen. Das Ventil 152 umfasst in den dargestellten Ausführungsbeispielen exemplarisch zwei Ventilglieder 154, 156, welche als Aktuatoren 158, 160 wirken können und welche als Quetschelemente in mindestens einem Quetschbereich 162 die Schlauchverbindung 144 quetschen können. Dabei können einer oder beide der Aktuatoren 158, 160 beweglich gelagert sein. Die Aktuatoren 158, 160 können beispielsweise über einen entsprechenden, in Figur 1A nicht dargestellten Antrieb beweglich gelagert sein. Die Aktuatoren 158, 160 wirken somit gemeinsam als Schlauchquetsche und sind eingerichtet, um einen Durchfluss des fluiden Mediums 112 durch den Quetschbereich 162 zu beeinflussen, beispielsweise freizugeben, zu drosseln oder zu unterbrechen.

Weiterhin umfasst die Medikationsvorrichtung in dem dargestellten Ausführungsbeispiel mindestens ein Messelement 164, welches eingerichtet ist, um eine relative Positionierung des verschiebbaren Elements 120 zu dem Behälter 116 zu erfassen. Diese Erfassung beruht in dem dargestellten Ausführungsbeispiel gemäß Figur 1A auf einer relativen Verschiebung des Behälters 116 innerhalb des Behältergehäuses 124, in welchem das verschiebbare Element 120 aufgrund der Lagerung auf dem Widerlager 132 ruht. Zu diesem Zweck weist das Behältergehäuse 124 eine Aussparung 166 auf. Durch diese Aussparung 166 greift ein mechanischer Fühler 168 ins Innere des Behältergehäuses 124 ein und legt auf dem Rand 118 des Behälters 116 auf. Über den Fühler 168 wird die Position des Randes 118 auf einen Messwertaufnehmer 170 übertragen, der beispielsweise die Position des Randes 118 in ein elektrisches Signal umwandeln kann. Beispielsweise kann die Position über mindestens einen Widerstand in einen Messwert umgesetzt werden und/oder kann über eine kapazitive oder induktive Kopplung in ein entsprechendes Signal umgewandelt werden.

Das Messelement 164 ist elektrisch mit mindestens einer Steuerung 172 verbunden. Zu diesem Zweck können die Steuerung 172 und das Messelement 164 als separate Elemente ausgestaltet sein. Alternativ kann die Steuerung 172 jedoch auch ganz oder teilweise in das Messelement 164 integriert sein und/oder das Messelement 164 kann ganz oder teilweise Bestandteil der Steuerung 172 sein.

Die Steuerung 172 ist eingerichtet, um derart mit dem Messelement 164 und dem Stellglied 150 zusammenzuwirken, dass die Abgabe des fluiden Mediums 112 durch die von dem Messelement 164 erfasste relative Positionierung des verschiebbaren Elements 120 zu dem Behälter 116 beeinflusst wird. Zu diesem Zweck kann die Steuerung 172 beispielsweise eingerichtet sein, um das Stellglied 150 zu steuern und/oder zu regeln. Diese Steuerung und/oder Regelung kann allgemein auf verschiedene Weisen ausgestaltet sein, welche in diesem oder auch in anderen Ausführungsbeispielen auch kombinierbar sind. So kann die Steuerung 172 beispielsweise eingerichtet sein, um eine Gesamtabgabemenge des fluiden Mediums 112 einzustellen. So kann die Steuerung 172 beispielsweise allgemein eingerichtet sein, um zunächst einen Durchfluss des fluiden Mediums 112 durch das Ventil 152 freizugeben, solange, bis eine vorgegebene Abgabemenge erreicht ist. Anschließend kann über das Ventil 152 der Durchfluss unterbrochen werden. Alternativ oder zusätzlich kann jedoch auch eine Rate der Abgabe des fluiden Mediums 112 eingestellt werden, beispielsweise eine Rate in Form eines abgegebenen Volumens und/oder in Form einer abgegebenen Masse pro Zeiteinheit. Verschiedene Ausgestaltungen sind möglich.

Die in Figur 1A dargestellte Medikationsvorrichtung 110 stellt somit ein äußerst einfach zu realisierendes, kostengünstiges und mit geringem Bauraum implementierbares Beispiel einer erfindungsgemäßen Medikationsvorrichtung 110 dar. Insbesondere die Konstruktion des Transport- und Transferbehältnisses 114 ermöglicht, wie oben ausgeführt, eine einfache Messung der Position des verschiebbaren Elements 120. Beispielsweise wird bei bekannten Insulinpumpen in vielen Fällen der innere Stopfen verschoben, dessen Position jedoch erheblich schwieriger zu erfassen wäre als die Erfassung der relativen Positionierung des Behälters 116 innerhalb des Behältergehäuses 124.

Auch die einfache Schlauchquetsche zur Durchflussregelung, welche hier exemplarisch vorgeschlagen wird, weist gegenüber bekannten Durchflussreglern zahlreiche Vorteile auf. Insbesondere kann als Schlauchverbindung 144 ein Silikonschlauch verwendet werden. Weiterhin ist die einfache Schlauchquetsche auch im Hinblick auf eine Insulinstabilität in der Regel unproblematisch.

Besondere Konstruktionsmerkmale können auch eine Doppelquetsche eigensicher machen, wie in den folgenden Ausführungsbeispielen unten noch näher erläutert wird. Dies kann beispielsweise bedeuten, dass nur in einem oszillierenden Betrieb des Stellglieds 150 ein Durchfluss möglich ist. Jede Endposition und auch eine energielose Zwischenposition blockieren den Durchfluss. Grundsätzlich könnte, alternativ oder zusätzlich, auch eine Schlauchpumpe einen sicheren Betrieb gewährleisten.

Weiterhin lässt sich, wie oben ausgeführt, das Ausführungsbeispiel gemäß Figur 1A äußerst bauraumgünstig ausgestalten. Zur Optimierung und/oder Reduktion der Baugröße besteht weiterhin die Möglichkeit, durch seitlich angeordnete Federn das in Figur 1A dargestellte Bauteil flacher auszugestalten. Die Dicke der Baugruppe wird dann in der Regel nur noch durch den Außendurchmesser des Behälters 116 und/oder des Transport- und Transferbehältnisses 114 bestimmt.

In Figur 2 ist ein weiteres Ausführungsbeispiel eines Stellglieds 150 dargestellt, welches, alternativ oder zusätzlich zu dem in Figur 1A dargestellten Stellglied 150, in einer erfindungsgemäßen Medikationsvorrichtung 110, beispielsweise gemäß Figur 1A, eingesetzt werden kann.

In diesem Ausführungsbeispiel weist das Stellglied 150 exemplarisch zwei Aktuatoren 158, 160 auf, welche, in Zusammenwirkung beispielsweise mit entsprechenden Widerlagern 174, 176, die Schlauchverbindung 144 in zwei beabstandeten Quetschbereichen 178, 180 quetschen können. Die Aktuatoren 158, 160 können dabei unabhängig voneinander betätigbar sein oder können auch, wie in Figur 2 dargestellt, mechanisch miteinander verbunden sein, beispielsweise über ein Ventilglied 182, welches in Figur 2 exemplarisch als Wippe ausgestaltet ist und drehbar um eine Achse 184 gelagert ist. Das Ventilglied 182 kann beispielsweise durch ein Federelement 186 und einen Ventilantrieb 188 angesteuert werden, beispielsweise derart, dass der Ventilantrieb 188 gegen eine Federkraft des Federelements 186 arbeitet und das Ventilglied 182 auf diese Weise wahlweise im Uhrzeigersinn oder gegen den Uhrzeigersinn um die Achse 184 gedreht werden kann.

Zwischen den beiden Aktuatoren 158, 160 kann ein Schlauchabschnitt 190 der Schlauchverbindung 144 angeordnet sein, welcher dehnbar ausgestaltet sein kann. Beim Ausströmen aus dem Behälter 116 durch die Schlauchverbindung 144 kann das fluide Medium 112 den Schlauchabschnitt 190 beispielsweise in einer Durchflussrichtung 192 passieren, wobei zunächst der Aktuator 158 als einlassseitiger Aktuator 194 und anschließend der Aktuator 160 als auslassseitiger Aktuator 196 passiert wird.

Das Stellglied 150 kann allgemein in folgender Weise eigensicher ausgestaltet sein. So kann beispielsweise das Stellglied 150 derart ausgestaltet sein, dass in keiner Ruheposition des Ventilglieds 182 ein Durchfluss durch den Schlauchabschnitt 190 ermöglicht ist. Beispielsweise kann die Positionierung des Stellglieds 150 derart erfolgen, dass in einer Ruheposition oder Gleichgewichtsposition der einlassseitige Aktuator 194 und er auslassseitige Aktuator 196 gleichermaßen einen Durchfluss sperren. Weiterhin können die Aktuatoren 194, 196 derart ausgestaltet sein, dass lediglich durch einen dieser Aktuatoren ein Durchfluss möglich ist, nicht hingegen durch beide dieser Aktuatoren 194, 196 gleichzeitig. Dies kann beispielsweise dadurch erfolgen, dass in der oben genannten Ruheposition beide Aktuatoren 194, 196 in den Quetschbereichen 178 und 180 quetschen. In einer ersten geöffneten Stellung kann beispielsweise dann der einlassseitige Aktuator 194 in Figur 2 nach oben bewegt werden, und der Durchfluss durch diesen Aktuator freigegeben werden. Durch die starre mechanische Verbindung zwischen den Aktuatoren 194, 196 wird dabei jedoch der auslassseitige Aktuator 196 nach unten bewegt und die Schlauchverbindung 144 in dem Quetschbereich 180 noch weiter gequetscht. Der Wandbereich der Schlauchverbindung 144 wirkt dabei als Reservoir, welches ein zusätzliches Quetschen aufnehmen kann, obwohl der auslassseitige Aktuator 196 bereits zuvor in einer geschlossenen Stellung war.

In dieser ersten Position kann, beispielsweise durch den Überdruck in dem Behälter 116, fluides Medium 112 durch den einlassseitigen Aktuator 194 in den Schlauchabschnitt 190 einströmen. Durch den Überdruck des fluiden Mediums 112 wird dieser Schlauchabschnitt 190, welcher dehnbar ausgestaltet ist, gedehnt. Diese erste Position oder Ventilstellung des Stellglieds 150 ist in Figur 2 dargestellt.

Anschließend kann das Ventilglied 182 im Gegenuhrzeigersinn um die Achse 184 gedreht werden. Der einlassseitige Aktuator 194 bewegt sich dabei in Figur 2 nach unten und bewirkt, in Zusammenwirkung mit dem Widerlager 174, eine Schließung dieses einlassseitigen Aktuators 194. Gleichzeitig wird der auslassseitige Aktuator 196 in Figur 2 nach oben bewegt und öffnet. Durch den in dem Schlauchabschnitt 190 herrschenden Überdruck, welcher durch Rückstellkräfte des Wandmaterials der Schlauchverbindung 144 zusätzlich unterstützt wird, strömt ein Teil des in dem Schlauchabschnitt 190 enthaltenen fluiden Mediums durch den auslassseitigen Aktuator 196 aus. Der Schlauchabschnitt 190, gemeinsam mit den Aktuatoren 194, 196, wirkt somit als Pumpsystem mit minimaler Durchflussrate.

Weiterhin kann das Stellglied 150, wie oben ausgeführt, derart ausgeführt werden, dass kein Zustand existiert, in welchem beide Aktuatoren 194, 196 offen sind, sodass ein ungehinderter Durchfluss oder überhaupt ein Durchfluss erfolgen kann. Auf diese oder auf andere Weise kann das Stellglied 150 eigensicher ausgestaltet werden, sodass beispielsweise bei einem Ausfall von Komponenten des Stellglieds 150 und/oder der Medikationsvorrichtung 110 kein ungehinderter Durchfluss des fluiden Mediums 112 auftreten kann. Beispielsweise kann in einem Ruhezustand, wie oben ausgeführt, das Ventil 152 derart ausgestaltet werden, dass beide Aktuatoren 194, 196 geschlossen sind.

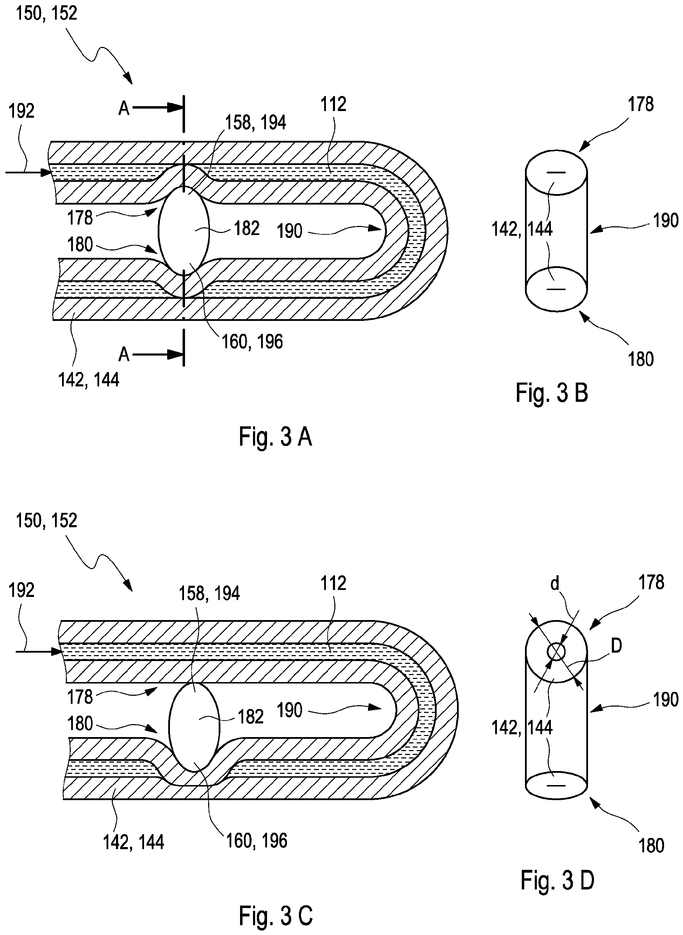

In den Figuren 3A-3F ist ein weiteres Ausführungsbeispiel eines Stellglieds eines Ventils 152 gezeigt, welches grundsätzlich analog zu dem Ausführungsbeispiel gemäß Figur 2 wirken kann. Dementsprechend kann in weiten Teilen bezüglich möglicher Ausgestaltungen auf die obige Beschreibung der Figur 2 verwiesen werden.

Wiederum weist das Stellglied 150 ein Ventil 152 mit einem Ventilglied 182 auf, dessen Enden als Aktoren 158 bzw. 160 wirken. Die Schlauchverbindung 144 ist in einer Schleife um dieses Ventilglied 182 herum geführt, sodass die Schlauchverbindung 144 das Ventilglied 182 zweimal passiert, nämlich an zwei Quetschbereichen 178 und 180. Wiederum wird durch eine Durchflussrichtung 192 des fluiden Mediums 112 durch einen Schlauchabschnitt 190, welcher vorzugsweise dehnbar ausgestaltet ist, zwischen den Aktoren 158, 160 der obere dieser Aktoren als einlassseitiger Aktor 194 definiert, und der jeweils untere dieser Aktoren als auslassseitiger Aktor 196.

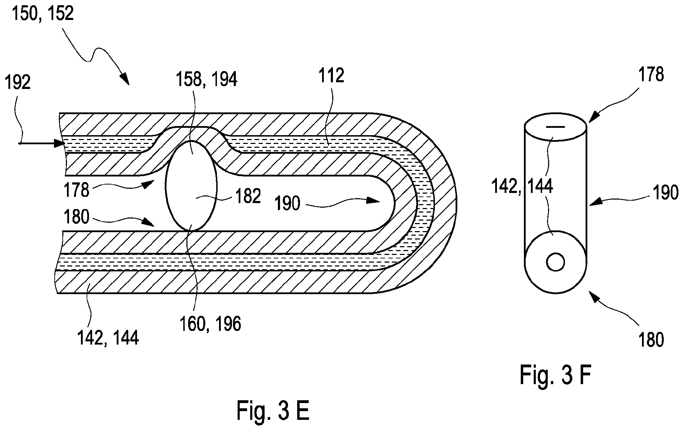

Dabei sind in den Figuren 3A-3F drei unterschiedliche Ventilstellungen des Ventilglieds 182 dargestellt. Das Ventilglied 182 kann sich in den Figuren vertikal auf- und abwärts bewegen, also jeweils beispielsweise senkrecht zu der Schlauchverbindung 144. Dabei zeigen die Figuren 3A und 3B eine erste Position, welche auch als Ruheposition oder als Neutralposition bezeichnet werden kann und in welcher das Ventilglied 182 weder nach oben noch nach unten ausgelenkt ist. Die Figuren 3C-3D beschreiben eine erste Position, analog zu der in Figur 2 dargestellten Position, in welcher der einlassseitige Aktuator 194 geöffnet, und der auslassseitige Aktuator 196 geschlossen ist, in dem das Ventilglied 182 in Figur 3C nach unten ausgelenkt ist. Diese Auslenkung kann beispielsweise wiederum durch einen Ventilantrieb 188 erfolgen, gegebenenfalls in Zusammenwirkung mit einem oder mehreren Federelementen 186, wobei in den Figuren 3A-3F der optionale mindestens eine Ventilantrieb 188 und das optionale mindestens eine Federelement 186 nicht dargestellt sind. In den Figuren 3E-3F ist hingegen eine Stellung beschrieben, in welcher das Ventilglied 182 nach oben ausgelenkt ist, sodass der einlassseitige Aktuator 194 geschlossen ist und der auslassseitige Aktuator 196 geöffnet ist. Allgemein zeigen jeweils die Figuren 3A, 3C und 3E die beschriebenen Positionen in einer Querschnittsdarstellung durch die Schlauchverbindung 144 von der Seite. Die Figuren 3B, 3D und 3F zeigen eine Schnittdarstellung senkrecht zur Schlauchverbindung 144 entlang der Schnittlinie A-A in Figur 3A. Das Ventilglied 182 ist in diesen Schnittdarstellungen nicht dargestellt.

Wie oben ausgeführt, kann das Ventil 152 eigensicher ausgestaltet sein, sodass in keiner der dargestellten Positionen, in welcher das Ventilglied 182 ruhen kann, ein vollständiger Durchfluss durch das Ventil 152 möglich ist. So sind in der in Figur 3A dargestellten Neutralstellung beide Ventile 194, 196 geschlossen. In der in Figur 3C dargestellten ersten Position ist das einlassseitige Ventil 194 geöffnet, das auslassseitige Ventil 196 jedoch geschlossen. In der in Figur 3E dargestellten zweiten Position ist das einlassseitige Ventil 194 geschlossen, das auslassseitige Ventil 196 hingegen geöffnet. Wie durch die Schnittdarstellungen in den Figuren 3B, 3D und 3F ersichtlich ist, kann dies beispielsweise dadurch erfolgen, dass in der in Figur 3A gezeigten Neutralstellung (siehe Schnitt in Figur 3B) beide Quetschbereiche 178, 180 gequetscht sind. Beim Auslenken des Ventilglieds 182 in die in Figur 3C dargestellte Position wird, wie in Figur 3D gezeigt, der zweite Quetschbereich 180 weiter gequetscht, wohingegen der erste Quetschbereich 178 entlastet und der Durchfluss dort ermöglicht wird. Bei der in Figur 3F dargestellten zweiten Position hingegen wird, ausgehend von der Neutralposition, der erste Quetschbereich 178 weiter gequetscht, wohingegen der zweite Quetschbereich 180 entlastet und der Durchfluss dort ermöglicht wird. Um, ausgehend von der bereits gequetschten Neutralposition in Figur 3B, ein weiteres Quetschen einer der beiden Quetschbereiche 178, 180 zu ermöglichen, ist es besonders vorteilhaft, ein Wandmaterial des Schlauchabschnitts 190 dehnbar zu gestalten. Um eine Dehnungsreserve bereitstellen zu können, ist es darüber hinaus vorteilhaft, einen Innendurchmesser d (siehe Figur 3D) des Schlauchabschnitts 190 kleiner zu gestalten als eine Wandstärke d des Schlauchabschnitts 190.

Bezüglich der Funktionsweise des Stellglieds 150 kann auf die obige Beschreibung der Figur 2 verwiesen werden. So kann zunächst bei geschlossenem auslassseitigem Aktuator 196 das fluide Medium 112 unter Druck in den Schlauchabschnitt 190 durch den geöffneten einlassseitigen Aktuator 194 einströmen. Anschließend kann der einlassseitige Aktuator 194 geschlossen und der auslassseitige Aktuator 196 geöffnet werden, sodass das fluide Medium, getrieben durch den Überdruck, aus dem Schlauchabschnitt 190 durch den auslassseitigen Aktuator 196 ausströmen kann. Wiederum kann auf diese Weise ein eigensicheres Stellglied realisiert werden, bei welchem in der in Figur 3A dargestellten Neutralstellung beide Aktuatoren 194, 196 geschlossen sind und bei welchem in keiner Ventilstellung, insbesondere in keiner statischen Ventilstellung, ein vollständiger Durchfluss des fluiden Mediums durch das Stellglied 150 ermöglicht wird.

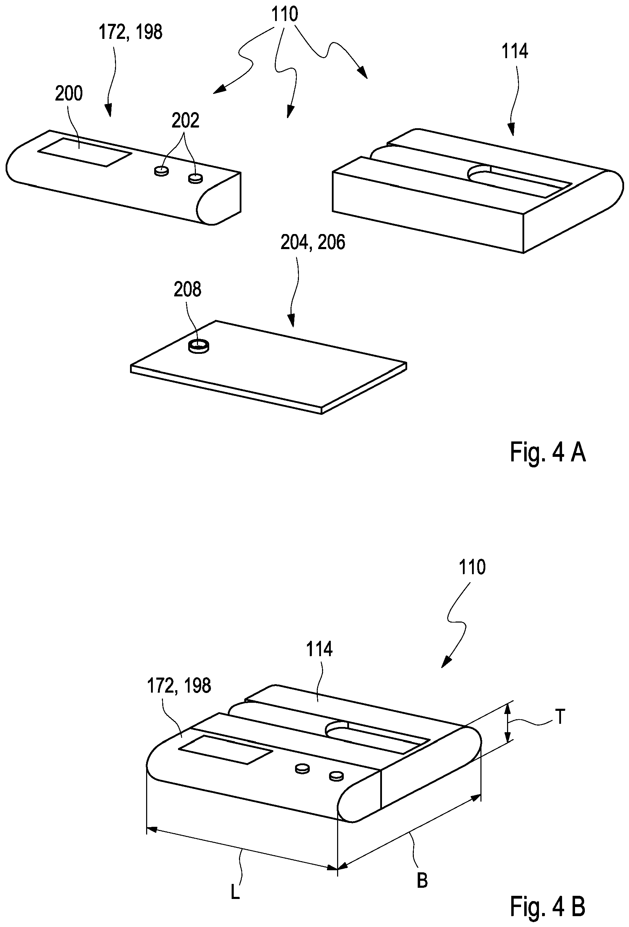

Ein besonderer Vorteil der erfindungsgemäßen Medikationsvorrichtung 110 in einer oder mehreren der dargestellten Ausgestaltungen besteht darin, dass die Medikationsvorrichtung mit besonders geringem Bauraum ausgestaltet werden kann und insbesondere sehr flach gestaltet werden kann. Insbesondere sind die oben beschriebenen Elemente des Transport-und Transferbehältnisses sowie der Steuerung 172 leicht trennbar, was dem Gedanken einer modularen Ausgestaltung zugute kommt. Ein Beispiel einer modular aufgebauten Medikationsvorrichtung 110 ist in den Figuren 4A und 4B dargestellt. Dabei zeigt Figur 4A verschiedene Module der Medikationsvorrichtung 110 in separater Darstellung, wohingegen Figur 4B die Medikationsvorrichtung 110 in einem zusammengesetzten Zustand der Module darstellt.

So kann die Medikationsvorrichtung 110 zunächst ein Transport- und Transferbehältnis 114 umfassen. Dieses kann beispielsweise analog zu der Darstellung in den Figuren 1A und 1B ausgestaltet sein. So kann das Transport- und Transferbehältnis 114 beispielsweise einen Behälter 116 umfassen und derart ausgestaltet sein, dass ein fluides Medium 112 (in den Figuren 4A und 4B nicht im Einzelnen dargestellt) mit einem Überdruck beaufschlagt ist, beispielsweise bereits seitens eines Herstellers. Alternativ oder zusätzlich kann jedoch auch mindestens ein Transport- und Transferbehältnis 114 vorgesehen sein, in welchem das fluide Medium 112 unter Normaldruck oder sogar unter Unterdruck aufgenommen ist. In diesem Fall kann der Transfer des fluiden Mediums 112 aus dem Behälter 116 heraus beispielsweise, wie unten noch näher exemplarisch ausgeführt wird, mittels mindestens einer Pumpe erfolgen.

Neben dem Transport- und Transferbehältnis 114, welches ein erstes Modul der modularen Medikationsvorrichtung 110 in den Figuren 4A und 4B bildet, umfasst die Medikationsvorrichtung 110 weiterhin ein Steuermodul 198. Dieses Steuermodul 198 kann beispielsweise die oben genannte Steuerung 172 umfassen, welche beispielsweise einen Durchfluss durch eine in den Figuren 4A und 4B nicht dargestellte Schlauchverbindung steuert und/oder regelt. Das Steuermodul 198 kann beispielsweise ein oder mehrere Anzeigenelemente 200 umfassen, beispielsweise ein oder mehrere Displays, beispielsweise um Durchflussraten und/oder Dosiermengen anzuzeigen. Weiterhin kann das Steuermodul 198 beispielsweise ein oder mehrere Bedienelemente 202 umfassen, beispielsweise Taster, Schalter oder ähnliche Bedienelemente. Die Steuerung 172 kann beispielsweise einen oder mehrere Mikroprozessoren enthalten und/oder andere Arten von Datenverarbeitungsgeräten, welche beispielsweise in dem Steuermodul 198 integriert sein können. Alternativ oder zusätzlich können auch ein oder mehrere elektronische Elemente vorgesehen sein, beispielsweise eine elektronische Regelung, beispielsweise um eine Durchflussrate einzustellen. Wiederum alternativ oder zusätzlich kann die Steuerung 172 auch ein oder mehrere Schalter umfassen, beispielsweise ein oder mehrere Endschalter, deren Schaltposition beispielsweise variabel einstellbar sein kann, sodass beispielsweise eine angegebene Gesamtmenge eingestellt werden kann.

Die Fluidverbindung 142 kann ganz oder teilweise ebenfalls in dem Modul des Transport-und Transferbehältnisses 114 enthalten sein oder kann ganz oder teilweise in einem separaten Modul enthalten sein. Selbiges gilt auch für das in den Figuren 4A und 4B ebenfalls nicht dargestellte mindestens eine Stellglied 150. So kann die Medikationsvorrichtung 110 beispielsweise weiterhin mindestens ein Fluidikmodul umfassen, welches beispielsweise die Fluidverbindung 142 und/oder das Stellglied 150 ganz oder teilweise aufnimmt. Weiterhin kann die Medikationsvorrichtung 110 ein Basismodul 204 umfassen. Dieses Basismodul 204 ist exemplarisch in Figur 4A in Form einer Basisplatte 206 dargestellt, auf welche das Steuermodul 198 und das Transport- und Transferbehältnis 114 aufgebracht werden können. Die Basisplatte 204 kann beispielsweise eine Öffnung 208 umfassen, durch welche hindurch die Fluidverbindung 142 geführt werden kann.

In Figur 4B ist die modulare Medikationsvorrichtung 110 in einem zusammengebauten Zustand dargestellt. Insgesamt kann die Medikationsvorrichtung 110 äußerst kompakt aufgebaut werden. Beispielsweise kann diese eine Länge L von 55 mm aufweisen, eine Breite B von 40 mm und eine Tiefe von 15 mm. Auch andere Dimensionierungen sind jedoch grundsätzlich möglich. Beispielsweise kann die Medikationsvorrichtung 110 gemäß Figur 4B für eine Abgabe von 1 ml eingerichtet sein.

Die modulare Medikationsvorrichtung 110 kann derart ausgestaltet sein, dass beispielsweise das Steuermodul 198 wiederverwendet werden kann. Die anderen Baugruppen, beispielsweise das Basismodul 204 und/oder das Transport- und Transferbehältnis 114, können als Einweg-Bauteile ausgestaltet werden. Durch die Verwendung eines vorgefüllten Behälters 116, beispielsweise eines vorgefüllten Insulinbehältnisses, kann eine Handhabung sehr einfach und sicher ausgestaltet werden, und eine blasenfreie Insulinabgabe kann garantiert werden. Alternativ oder zusätzlich besteht auch die Möglichkeit, den Behälter 116 durch einen Benutzer manuell zu befüllen.

Zur Vorbereitung der Medikationsvorrichtung 110 für den Routinebetrieb kann beispielsweise zunächst ein Infusionsset in das Basismodul 204 eingelegt werden. Alternativ oder zusätzlich kann das Infusionsset auch ganz oder teilweise Bestandteil des Basismoduls 204 sein. Das Infusionsset kann beispielsweise die Fluidverbindung 142 oder Teile derselben umfassen, beispielsweise eine Kanüle zur Perforation einer Haut eines Benutzers. Weiterhin kann dieses Infusionsset auch beispielsweise die Schlauchverbindung 144 oder Teile desselben und/oder das Perforationselement 122 oder Teile desselben und/oder das Stellglied 150 oder Teile desselben umfassen. Alternativ oder zusätzlich können auch Teile der genannten Elemente außerhalb des Infusionssets angeordnet sein, beispielsweise innerhalb oder außerhalb des Basismoduls 204. Beispielsweise kann die Basisplatte 206 mindestens ein Pflaster und/oder mindestens eine Klebefläche aufweisen, mittels derer die Basisplatte 206 und/oder das Basismodul 204 ganz oder teilweise auf die Haut des Benutzers aufgeklebt werden können. Beispielsweise kann die Basisplatte 206 mitsamt dem Infusionsset und/oder Teilen desselben, beispielsweise der Kanüle zur Perforation der Haut des Benutzers, auf die Haut aufgeklebt werden. Für eine Reinigung des Benutzers, beispielsweise zum Baden oder zum Duschen des Benutzers, kann beispielsweise das Basismodul 204 zerlegt werden, wobei beispielsweise die Basisplatte 206 mitsamt der Kanüle auf bzw. in der Haut verbleiben kann, wohingegen beispielsweise das Steuermodul 198 und/oder das Transport- und Transferbehältnis 118 von der Basisplatte 206 getrennt und abgenommen werden können. Die Basisplatte 206 mitsamt des Infusionssets kann in regelmäßigen oder unregelmäßigen Abständen ausgetauscht werden, beispielsweise einmal wöchentlich.

Das Infusionsset, beispielsweise mit der Kanüle zur Perforation der Haut des Benutzers, kann beispielsweise ganz oder teilweise Bestandteil der Basisplatte 206 sein oder fest oder reversibel mit der Basisplatte 206 verbunden oder verbindbar sein. So kann beispielsweise zwischen dem Infusionsset und der Basisplatte 206 eine Steckverbindung, eine Schraubverbindung oder eine Bajonettverbindung herstellbar sein. Auch andere Ausgestaltungen sind möglich.

In einem weiteren Arbeitsschritt kann das Transport- und Transferbehältnis 114 montiert werden, wobei beispielsweise das Perforationselement 122 das verschiebbare Element 120 perforiert. Diese Perforation kann beispielsweise während eines Einschiebens des Transport- und Transferbehältnisses 114 in das Basismodul 204 automatisch erfolgen.

Anschließend kann beispielsweise ein Funktionstest und eine optionale Erstentlüftung zu erfolgen, gefolgt von einer Injektion des fluiden Mediums 112 in ein Körpergewebe, beispielsweise einer subkutanen Insulin-Injektion.

Besonders vorteilhaft ist zu vermerken, dass nach dem Entleeren des Behälters 116 ein Austausch des Transport- und Transferbehältnisses 114 auf einfache Weise erfolgen kann. Nach dem Austausch des Transport- und Transferbehältnisses 114 ist die Medikationsvorrichtung 114 vorzugsweise unmittelbar wieder betriebsbereit, ohne dass weitere Arbeitsschritte erforderlich sind, wie beispielsweise ein Entlüften. Die beschriebenen Ideen eignen sich auch zum Aufbau einer Patch-Pumpe. Auch diese würde grundsätzlich beispielsweise drei oder vier Baugruppen oder Module umfassen. Da grundsätzlich kein Motor und kein großer Energiebedarf erforderlich sind, können beispielsweise die Größe und das Gewicht der Medikationsvorrichtung optimiert werden. Im Vergleich zu herkömmlichen Pumpen wie beispielsweise Insulinpumpen sollten Volumen- und Gewichtseinsparungen von beispielsweise 25% möglich seien.

Wie oben beschrieben, lassen sich die Module und deren Funktionen auf verschiedene Weisen gruppieren. So lässt sich beispielsweise eine Injektionsvorrichtung mit dem Basismodul 204, beispielsweise der Basisplatte 206, integrieren. Die Steuerung 172 lässt sich in das Steuermodul 198 integrieren. Weiterhin lassen sich auch andere elektronische Komponenten sowie das Messelement 164 vollständig oder teilweise in das Steuermodul 198 integrieren. Weiterhin kann das Steuermodul 198, wie oben ausgeführt, eine oder mehrere Datenverarbeitungsvorrichtungen umfassen. So kann beispielsweise in dem Steuermodul 198 auch mindestens ein sogenannter Datamanager vorgesehen sein, welcher beispielsweise auch mindestens eine Datenbank und/oder mindestens ein Datenbankprogramm umfassen kann. Die Medikationsvorrichtung 110 kann auch beispielsweise mindestens eine Fernbedienung aufweisen oder mit mindestens einer Fernbedienung zusammenwirken, welche in den Figuren nicht dargestellt ist und welche beispielsweise mit der Steuerung 172, beispielsweise dem Steuermodul 198, drahtlos oder auch drahtgebunden verbunden sein kann, so dass beispielsweise eine Fernsteuerung einer Funktion der Medikationsvorrichtung 110 möglich ist. Beispielsweise kann eine drahtlose Verbindung in Form einer Funkverbindung oder einer anderen Verbindung über elektromagnetische Wellen zu einer Fernbedienung vorgesehen sein.

Weiterhin lassen sich, wie oben ausgeführt, auch Teile der Fluidverbindung 172 ganz oder teilweise in das Transport- und Transferbehältnis 114 integrieren. Auf diese Weise lassen sich die Fluidverbindung 172 oder Teile derselben mit dem Transport- und Transferbehältnis 114 beispielsweise zu einem Einwegsystem zusammenfassen.

Bei einer derartigen Zusammenfassung der Komponenten lässt sich eine Betriebsbereitschaft beispielsweise derart herstellen, dass zunächst das Transport- und Transferbehältnis 114 mit dem Steuermodul 198 verbunden wird. In einem weiteren Arbeitsschritt wird beispielsweise die Basisplatte 206 auf einer Körperoberfläche fixiert. Weiterhin werden das Transport- und Transferbehältnis 114, gemeinsam mit dem Steuermodul 198, auf der Basisplatte 206 aufgesetzt, und eine Betriebsbereitschaft wird hergestellt.

Insgesamt lässt sich auf diese Weise eine nutzerfreundliche Medikationsvorrichtung 110, insbesondere eine nutzerfreundliche Insulinpumpe, gestalten, bei welcher günstige Einweg-Baugruppen das manuelle Füllen und ein sogenanntes Priming (Erstbefüllung) ersetzen können. Steriltechnische Vorteile und gute Produktstabilität können durch die Verwendung von einfachen, bekannten Materialien gewährleistet werden. Weiterhin lässt sich ein blasenfreier Behälterwechsel gewährleisten. Durch die Option, eine eigensichere Konstruktion des Stellglieds 150, insbesondere des Ventils 152, zu gewährleisten, lässt sich die Medikationsvorrichtung mit hoher Sicherheit und minimiertem Risiko für einen Benutzer ausgestalten.

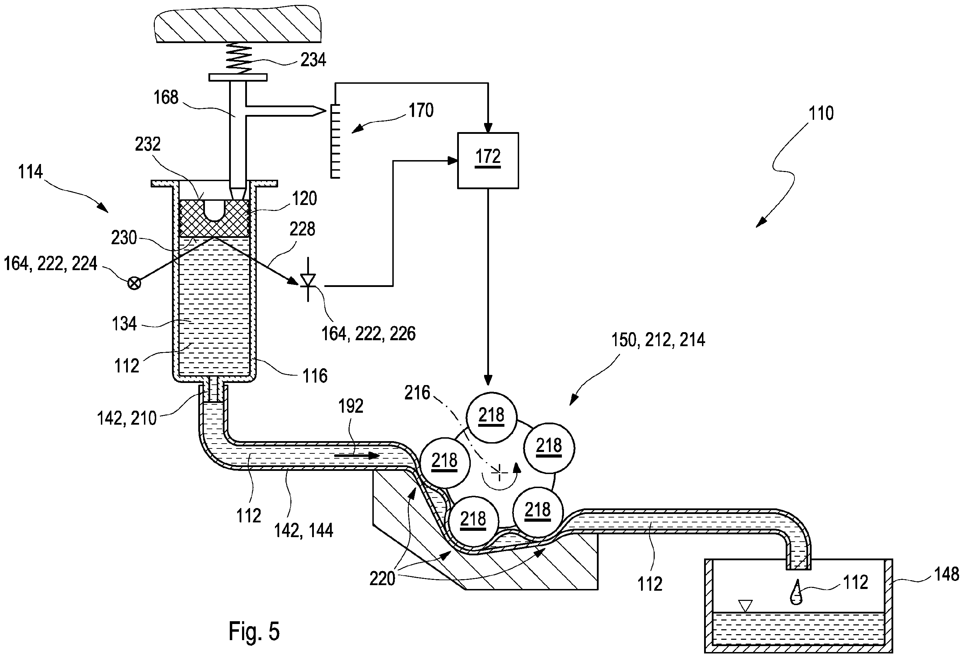

Die in Figur 1 dargestellte Medikationsvorrichtung kann in verschiedener Weise modifiziert werden, wobei die Modifikationen einzeln, in Gruppen oder insgesamt durchführbar sind. Eine mögliche Ausgestaltung von Modifikationen ist in Figur 5 dargestellt, in welcher eine alternative Medikationsvorrichtung 110 in einer zu Figur 1A analogen Darstellung gezeigt ist.

So umfasst die Medikationsvorrichtung 110 gemäß Figur 5 zunächst wiederum ein Transport- und Transferbehältnis 114 mit einem Behälter 116 zur Aufnahme eines fluiden Mediums 112. Dieser Behälter 116 ist wiederum durch ein verschiebbares Element 120 verschlossen, beispielsweise wiederum durch einen Stopfen, wobei das verschiebbare Element 120 verschiebbar in dem Behälter 116 gelagert ist. Die Herstellung einer Fluidverbindung zu einem Innenraum 134 des Behälters 116 kann, wie oben ausgeführt, durch das verschiebbare Element 120 hindurch erfolgen. Alternativ oder zusätzlich kann der Behälter 116 jedoch auch, wie in Figur 5 gezeigt, eine andere Art von Fluidauslass aufweisen, beispielsweise einen Stutzen 210, welcher Bestandteil einer Fluidverbindung 142 sein kann. Diese Fluidverbindung kann wiederum, wie auch im Ausführungsbeispiel gemäß Figur 1A, eine Schlauchverbindung 144 umfassen, beispielsweise wiederum einen Silikonschlauch. Das fluide Medium 112 in dem Behälter 116 ist nicht notwendigerweise mit einem Überdruck beaufschlagt. Um das fluide Medium 112 aus dem Behälter 116 zu transferieren, kann beispielsweise auch eine Saugkraft verwendet werden, welche beispielsweise mittels einer oder mehrerer Pumpen 212 bereitgestellt werden kann. Die in Figur 5 dargestellte Pumpe 212 stellt ein weiteres, alternativ oder zusätzlich zu dem in Figur 1A gezeigten Ventil 152 einsetzbares Ausführungsbeispiel eines Stellglieds 150 dar, über welches die Abgabe des fluiden Mediums 112 beeinflussbar ist. Im dargestellten Ausführungsbeispiel kann diese Pumpe 212 beispielsweise als Peristaltikpumpe 214 ausgestaltet sein, beispielsweise als Peristaltikpumpe 214 mit um eine Achse 216 rotierenden Aktuatoren 218. Diese Aktuatoren 218 können beispielsweise in wandernden Quetschbereichen 220 auf die Schlauchverbindung 144 einwirken, sodass das fluide Medium 112 in einer Durchflussrichtung 192 durch die Schlauchverbindung 144 transportiert wird und beispielsweise in ein Gefäß 148 befördert wird.

Weiterhin ist in Figur 5 eine Option dargestellt, welche unabhängig von den übrigen genannten Optionen einsetzbar ist und welche insbesondere bei Transport- und Transferbehältnissen 114 ohne Überdruck-Beaufschlagung des fluiden Mediums 112 eingesetzt werden kann. Bei dieser Ausgestaltung wird, im Gegensatz zur Erfassung der Position des beweglichen Behälters 116 in dem Ausführungsbeispiel gemäß Figur 1A, unmittelbar eine Position des verschiebbaren Elements 120 gemessen. Auch diesbezüglich sind zwei Optionen gezeigt. So kann einerseits ein Messelement 164 in Form eines optischen Messwertaufnehmers 222 vorgesehen sein, mit einer Lichtquelle 224 und einem Detektor 226. Beispielsweise über die Reflexion eines Lichtstrahls 228 kann dabei unmittelbar und direkt eine Position einer Grenzfläche 230 zwischen dem verschiebbaren Element 120, beispielsweise dem verschiebbaren Stopfen, und dem fluiden Medium 112 im Innenraum 134 des Behälters 116 erfasst werden.

Alternativ oder zusätzlich kann die Position des verschiebbaren Elements 120 relativ zu dem Behälter 116 auch auf andere Weise erfasst werden. So kann beispielsweise auf einer Rückseite 232 des verschiebbaren Elements 120, außerhalb des Innenraums 134, ein Fühler 168 vorgesehen sein, welcher beispielsweise über ein Federelement 234 gegen die Rückseite 232 gepresst werden kann. Der Fühler 168 kann beispielsweise mit einem Messwertaufnehmer 170 gekoppelt sein, sodass wiederum, wie auch bei dem optischen Messwertaufnehmer 222, ein Signal an die Steuerung 172 bereitgestellt werden kann, mittels dessen dann beispielsweise wiederum das Stellglied 150, unabhängig von dessen Ausgestaltung, gesteuert und/oder geregelt werden kann.

**Bezugszeichenliste**

| | | | |
|---|---|---|---|
| 110 | Medikationsvorrichtung | 178 | Quetschbereich |
| 112 | fluides Medium | 180 | Quetschbereich |
| 114 | Transport-und Transferbehältnis | 182 | Ventilglied |
| 116 | Behälter | 184 | Achse |
| 118 | Rand | 186 | Federelement |
| 120 | verschiebbares Element | 188 | Ventilantrieb |
| 122 | Perforationselement | 190 | Schlauchabschnitt |
| 124 | Behältergehäuse | 192 | Durchflussrichtung |
| 126 | Energieeinheit | 194 | einlassseitiger Aktuator |
| 128 | Energiespeicher | 196 | auslassseitiger Aktuator |
| 130 | Federelement | 198 | Steuermodul |
| 132 | Widerlager | 200 | Anzeigenelement |
| 134 | Innenraum | 202 | Bedienelement |
| 136 | Abdichtung | 204 | Basiselement |
| 138 | Hohlraum | 206 | Basisplatte |
| 140 | Führung | 208 | Öffnung |
| 142 | Fluidverbindung | 210 | Stutzen |
| 144 | Schlauchverbindung | 212 | Pumpe |
| 146 | Abgabestelle | 214 | Peristaltikpumpe |
| 148 | Gefäß | 216 | Achse |
| 150 | Stellglied | 218 | Aktuatoren |
| 152 | Ventil | 220 | Quetschbereich |
| 154 | Ventilglieder | 222 | optischer Messaufnehmer |
| 156 | Ventilglieder | 224 | Lichtquelle |
| 158 | Aktuator | 226 | Detektor |
| 160 | Aktuator | 228 | Lichtstrahl |
| 162 | Quetschbereich | 230 | Grenzfläche |
| 164 | Messelement | 232 | Rückseite |
| 166 | Aussparung | 234 | Federelement |
| 168 | Fühler | | |
| 170 | Messwertaufnehmer | | |
| 172 | Steuerung | | |
| 174 | Widerlager | | |
| 176 | Widerlager | | |

## Patentansprüche

1. Medikationsvorrichtung (110) zur dosierten Abgabe eines fluiden Mediums (112), insbesondere eines fluiden Therapeutikums und/oder Diagnostikums, insbesondere Insulinpumpe, umfassend:
a) mindestens ein Transport- und Transferbehältnis (114), wobei das Transport- und Transferbehältnis (114) mindestens einen Behälter (116) zur Aufnahme des fluiden Mediums (112) aufweist, wobei der Behälter (116) mindestens ein verschiebbares Element (120) aufweist, wobei das verschiebbare Element (120) relativ zu dem Behälter (116) beweglich gelagert ist und wobei eine relative Positionierung des verschiebbaren Elements (120) zu dem Behälter (116) einen zur Aufnahme des fluiden Mediums (112) zur Verfügung stehenden Innenraum (134) des Behälters (116) bestimmt,
b) mindestens ein Messelement (164), wobei das Messelement (164) eingerichtet ist, um die relative Positionierung des verschiebbaren Elements (120) zu dem Behälter (116) zu erfassen, und
c) mindestens ein Stellglied (150) zur Beeinflussung einer Abgabe des fluiden Mediums (112) über mindestens eine Fluidverbindung (142) zu dem Innenraum (134),
wobei das Messelement (164) und das Stellglied (150) derart zusammenwirken, dass die Abgabe des fluiden Mediums (112) durch die von dem Messelement (164) erfasste relative Positionierung beeinflusst wird, **dadurch gekennzeichnet, dass** die Medikationsvorrichtung (110) mindestens ein Gehäuse (124) aufweist,
wobei der Behälter (116) beweglich in dem Gehäuse (124) aufgenommen ist und
wobei das verschiebbare Element (120) relativ zu dem Gehäuse (124) fixiert ist, wobei das Messelement (164) eingerichtet ist, um eine relative Positionierung des Behälters (116) in dem Gehäuse (124) zu erfassen.

2. Medikationsvorrichtung (110) nach dem vorhergehenden Anspruch, wobei die Medikationsvorrichtung (110) eingerichtet ist, um das verschiebbare Element (120) gegen ein Widerlager (132) zu pressen, so dass das verschiebbare Element (120) in den Innenraum (134) hinein gedrückt wird.

3. Medikationsvorrichtung (110) nach dem vorhergehenden Anspruch, wobei die Fluidverbindung (142) durch das Widerlager (132) hindurch erfolgt.

4. Medikationsvorrichtung (110) nach einem der vorhergehenden Ansprüche, wobei die Fluidverbindung (142) das verschiebbare Element (120) durchdringt.

5. Medikationsvorrichtung (110) nach einem der vorhergehenden Ansprüche, wobei das Messelement (164) eingerichtet ist, um mindestens ein Messsignal entsprechend der relativen Positionierung zu erzeugen, wobei die Medikationsvorrichtung (110) mindestens eine Steuerung (172) aufweist, wobei die Steuerung (172) eingerichtet ist, um das Stellglied (150) mittels des Messsignals zu steuern und/oder zu regeln.

6. Medikationsvorrichtung (110) nach einem der vorhergehenden Ansprüche, wobei das verschiebbare Element (120) einen relativ zu dem Behälter (116) verschiebbaren Stopfen umfasst, wobei das Messelement (164) eingerichtet ist, um eine Verschiebung des Stopfens in dem Behälter (116), insbesondere eine lineare Verschiebung, zu erfassen.

7. Medikationsvorrichtung (110) nach einem der vorhergehenden Ansprüche, wobei das Messelement (164) eingerichtet ist, um mindestens eine Position mindestens einer Grenzfläche (230) zwischen dem verschiebbaren Element (120) und dem fluiden Medium (112) zu erfassen.

8. Medikationsvorrichtung (110) nach einem der vorhergehenden Ansprüche, wobei die Medikationsvorrichtung (110) eingerichtet ist, um das verschiebbare Element (120) und/oder den Behälter (116) derart mit einer Kraft zu beaufschlagen, dass das fluide Medium (112) unter einem erhöhten Druck steht.

9. Medikationsvorrichtung (110) nach einem der vorhergehenden Ansprüche, wobei die Medikationsvorrichtung (110) mindestens eine Energieeinheit (126) aufweist, wobei die Energieeinheit (126) eingerichtet ist, um das fluide Medium (112) in dem Behälter (116) mit einem Überdruck zu beaufschlagen, wobei die Energieeinheit (126) Bestandteil des Transport- und Transferbehältnisses (114) ist.

10. Medikationsvorrichtung (110) nach einem der vorhergehenden Ansprüche, wobei das Stellglied (150) mindestens ein Ventil (152) aufweist, wobei das Ventil (152) eingerichtet ist, um einen Durchfluss des fluiden Mediums (112) durch die Fluidverbindung (142) zu beeinflussen, insbesondere wahlweise zu unterbrechen oder freizugeben.

11. Medikationsvorrichtung (110) nach dem vorhergehenden Anspruch, wobei das Ventil (152) mindestens zwei geschlossene Ventilstellungen aufweist, wobei ein Durchfluss des fluiden Mediums (112) unterbrochen ist, wenn das Ventil (152) in einer der geschlossenen Ventilstellungen ist.

12. Medikationsvorrichtung (110) nach dem vorhergehenden Anspruch, wobei die geschlossenen Ventilstellungen Endstellungen einer Bewegung mindestens eines Ventilglieds (154, 156; 182) des Ventils (152) umfassen, wobei sich das Ventilglied (154, 156; 182) zwischen den Ventilstellungen bewegen kann.

13. Medikationsvorrichtung (110) nach einem der drei vorhergehenden Ansprüche, wobei sich das Ventil (152) in einer Ruhestellung, in welcher keine Kraft auf ein Ventilglied (154, 156; 182) des Ventils (152) ausgeübt wird, in einer geschlossenen Ventilstellung befindet, in welcher der Durchfluss des fluiden Mediums (112) durch die Fluidverbindung (142) unterbrochen ist.

14. Medikationsvorrichtung (110) nach einem der vier vorhergehenden Ansprüche, wobei das Ventil (152) mindestens ein Ventilglied (154, 156; 182) aufweist, wobei das Ventilglied (154, 156; 182) in mindestens zwei statische Ventilpositionen eingestellt werden kann, wobei in allen statischen Ventilpositionen ein Durchfluss des fluiden Mediums (112) durch die Fluidverbindung (142) unterbrochen ist.

15. Medikationsvorrichtung (110) nach einem der vorhergehenden Ansprüche, wobei das Stellglied (150) mindestens eine Pumpe (212) aufweist.

16. Medikationsvorrichtung (110) nach einem der vorhergehenden Ansprüche, wobei das Stellglied (150) mindestens zwei Aktuatoren (158, 160; 194, 196) umfasst, wobei die Fluidverbindung (142) mindestens einen Schlauchabschnitt (190) umfasst, wobei die Aktuatoren (158, 160; 194, 196) eingerichtet sind, um den Schlauchabschnitt (190) in mindestens zwei verschiedenen Quetschbereichen (178, 180; 220) zu quetschen, wobei zwischen den Aktuatoren (158, 160; 194, 196) ein dehnbarer Bereich des Schlauchabschnitts (190) angeordnet ist, wobei die Aktuatoren (158, 160; 194, 196) einen einlassseitigen Aktuator (194) und einen auslassseitigen Aktuator (196) umfassen, wobei die Medikationsvorrichtung (110) eingerichtet ist, um bei geöffnetem einlassseitigem Aktuator (194) und geschlossenem auslassseitigem Aktuator (196) fluides Medium (112) unter Druck in den dehnbaren Bereich zwischen den Aktuatoren (194, 196) einzulassen, wobei der dehnbare Bereich gedehnt wird, wobei die Medikationsvorrichtung (110) weiterhin eingerichtet ist, um anschließend den einlassseitigen Aktuator (194) zu schließen und den auslassseitigen Aktuator (196) zu öffnen, wobei der dehnbare Bereich entspannt wird und ein Teil des fluiden Mediums (112) aus dem dehnbaren Bereich ausströmt.

17. Medikationsvorrichtung (110) nach einem der vorhergehenden Ansprüche, wobei die Medikationsvorrichtung (110) modular aufgebaut ist, wobei die Medikationsvorrichtung (110) mindestens ein Steuermodul mit einer Steuerung (172) umfasst, wobei das Transport- und Transferbehältnis (114) mindestens ein weiteres Modul der Medikationsvorrichtung (110) bildet, wobei das Transport- und Transferbehältnis (114) austauschbar ausgestaltet ist.

18. Verfahren zur dosierten Abgabe eines fluiden Mediums (112), insbesondere eines fluiden Therapeutikums und/oder Diagnostikums, insbesondere unter Verwendung einer Medikationsvorrichtung (110) nach einem der vorhergehenden Ansprüche,
- wobei mindestens ein Transport- und Transferbehältnis (114) verwendet wird, wobei das Transport- und Transferbehältnis (114) mindestens einen Behälter (116) zur Aufnahme des fluiden Mediums (112) aufweist, wobei der Behälter (116) mindestens ein verschiebbares Element (120) aufweist, wobei das verschiebbare Element (120) relativ zu dem Behälter (116) beweglich gelagert wird und wobei eine relative Positionierung des verschiebbaren Elements (120) zu dem Behälter (116) einen zur Aufnahme des fluiden Mediums (112) zur Verfügung stehenden Innenraum (134) des Behälters (116) bestimmt,
- wobei mittels mindestens eines Messelements (164) die relative Positionierung des verschiebbaren Elements (120) zu dem Behälter (116) erfasst wird, wobei der Behälter (116) beweglich in einem Gehäuse (124) einer Medikationsvorrichtung (110) aufgenommen wird und wobei das verschiebbare Element (120) relativ zu dem Gehäuse (124) fixiert wird, wobei das Messelement (164) eine relative Positionierung des Behälters (116) in dem Gehäuse (124) erfasst,
- wobei weiterhin mindestens ein Stellglied (150) zur Beeinflussung einer Abgabe des fluiden Mediums (112) über mindestens eine Fluidverbindung (142) zu dem Innenraum (134) verwendet wird,
- wobei das Messelement (164) und das Stellglied (150) derart zusammenwirken, dass die Abgabe des fluiden Mediums (112) durch die von dem Messelement (164) erfasste relative Positionierung des verschiebbaren Elements (120) zu dem Behälter (116) beeinflusst wird.

19. Verwendung einer Medikationsvorrichtung (110) nach einem der vorhergehenden, eine Medikationsvorrichtung (110) betreffenden Ansprüche zur Dosierung eines Therapeutikums und/oder Diagnostikums, insbesondere Insulin.

## Claims

1. Medication device (110) for the dosed dispensing of a fluidic medium (112), in particular a fluidic therapeutic and/or diagnostic agent, in particular an insulin pump, comprising:
a) at least one transporting and transferring container (114), the transporting and transferring container (114) having at least one reservoir (116) for receiving the fluidic medium (112), the reservoir (116) having at least one displaceable element (120), the displaceable element (120) being mounted movably in relation to the reservoir (116) and a relative positioning of the displaceable element (120) in relation to the reservoir (116) determining an interior space (134) of the reservoir (116) available for receiving the fluidic medium (112),
b) at least one measuring element (164), the measuring element (164) being designed to sense the relative positioning of the displaceable element (120) in relation to the reservoir (116), and
c) at least one adjusting element (150) for influencing a dispensing of the fluidic medium (112) by way of at least one fluid connection (142) to the interior space (134),
the measuring element (164) and the adjusting element (150) interacting in such a way that the dispensing of the fluidic medium (112) is influenced by the relative positioning sensed by the measuring element (164),
**characterized in that**
the medication device (110) has at least one housing (124),
the reservoir (116) being movably accommodated in the housing (124) and the displaceable element (120) being fixed in relation to the housing (124), the measuring element (164) being designed to sense a relative positioning of the reservoir (116) in the housing (124).

2. Medication device (110) according to the preceding claim, the medication device (110) being designed to press the displaceable element (120) against an abutment (132), so that the displaceable element (120) is forced into the interior space (134).

3. Medication device (110) according to the preceding claim, the fluid connection (142) taking place through the abutment (132).

4. Medication device (110) according to one of the preceding claims, the fluid connection (142) penetrating through the displaceable element (120).

5. Medication device (110) according to one of the preceding claims, the measuring element (164) being designed to generate at least one measuring signal corresponding to the relative positioning, the medication device (110) having at least one controller (172), the controller (172) being designed to control the adjusting element (150) in an open-loop and/or closed-loop mode by means of the measuring signal.

6. Medication device (110) according to one of the preceding claims, the displaceable element (120) comprising a stopper which is displaceable in relation to the reservoir (116), the measuring element (164) being designed to sense a displacement of the stopper in the reservoir (116), in particular a linear displacement.

7. Medication device (110) according to one of the preceding claims, the measuring element (164) being designed to sense at least one position of at least one interface (230) between the displaceable element (120) and the fluidic medium (112).

8. Medication device (110) according to one of the preceding claims, the medication device (110) being designed to subject the displaceable element (120) and/or the reservoir (116) to a force in such a way that the fluidic medium (112) is under an increased pressure.

9. Medication device (110) according to one of the preceding claims, the medication device (110) having at least one energy unit (126), the energy unit (126) being designed to subject the fluidic medium (112) in the reservoir (116) to a positive pressure, the energy unit (126) being a component part of the transporting and transferring container (114) .

10. Medication device (110) according to one of the preceding claims, the adjusting element (150) having at least one valve (152), the valve (152) being designed to influence, in particular optionally to interrupt or allow, a flow of the fluidic medium (112) through the fluid connection (142) .

11. Medication device (110) according to the preceding claim, the valve (152) having at least two closed valve positions, a flow of the fluidic medium (112) being interrupted when the valve (152) is in one of the closed valve positions.

12. Medication device (110) according to the preceding claim, the closed valve positions comprising end positions of a movement of at least one valve element (154, 156; 182) of the valve (152), the valve element (154, 156; 182) being able to move between the valve positions.

13. Medication device (110) according to one of the three preceding claims, the valve (152) in a rest position, in which no force is exerted on a valve element (154, 156; 182) of the valve (152), being in a closed valve position, in which the flow of the fluidic medium (112) through the fluid connection (142) is interrupted.

14. Medication device (110) according to one of the four preceding claims, the valve (152) having at least one valve element (154, 156; 182), the valve element (154, 156; 182) being able to be set in at least two static valve positions, a flow of the fluidic medium (112) through the fluid connection (142) being interrupted in all the static valve positions.

15. Medication device (110) according to one of the preceding claims, the adjusting element (150) having at least one pump (212).

16. Medication device (110) according to one of the preceding claims, the adjusting element (150) comprising at least two actuators (158, 160; 194, 196), the fluid connection (142) comprising at least one hose portion (190), the actuators (158, 160; 194, 196) being designed to pinch the hose portion (190) in at least two different pinching regions (178, 180; 220), a stretchable region of the hose portion (190) being arranged between the actuators (158, 160; 194, 196), the actuators (158, 160; 194, 196) comprising an actuator on the inlet side (194) and an actuator on the outlet side (196), the medication device (110) being designed to admit fluidic medium (112) under pressure into the stretchable region between the actuators (194, 196) when the actuator on the inlet side (194) is open and the actuator on the outlet side (196) is closed, the stretchable region being stretched, the medication device (110) also being designed to subsequently close the actuator on the inlet side (194) and open the actuator on the outlet side (196), the stretchable region being relaxed and part of the fluidic medium (112) flowing out of the stretchable region.

17. Medication device (110) according to one of the preceding claims, the medication device (110) being of a modular structure, the medication device (110) comprising at least one control module with a controller (172), the transporting and transferring container (114) forming at least one further module of the medication device (110), the transporting and transferring container (114) being of an exchangeable configuration.

18. Method for the dosed dispensing of a fluidic medium (112), in particular a fluidic therapeutic and/or diagnostic agent, in particular using a medication device (110) according to one of the preceding claims,
- at least one transporting and transferring container (114) being used, the transporting and transferring container (114) having at least one reservoir (116) for receiving the fluidic medium (112), the reservoir (116) having at least one displaceable element (120), the displaceable element (120) being mounted movably in relation to the reservoir (116) and a relative positioning of the displaceable element (120) in relation to the reservoir (116) determining an interior space (134) of the reservoir (116) available for receiving the fluidic medium (112),
- the relative positioning of the displaceable element (120) in relation to the reservoir (116) being sensed by means of at least one measuring element (164), the reservoir (116) being movably accommadated in a housing (124) of a medication device (110) and the displaceable element (120) being fixed in relation to the housing (124), the measuring element (164) sensing a relative positioning of the reservoir (116) in the housing (124),
- at least one adjusting element (150) for influencing a dispensing of the fluidic medium (112) by way of at least one fluid connection (142) to the interior space (134) also being used,
- the measuring element (164) and the adjusting element (150) interacting in such a way that the dispensing of the fluidic medium (112) is influenced by the relative positioning, sensed by the measuring element (164), of the displaceable element (120) in relation to the reservoir (116).

19. Use of the medication device (110) according to one of the preceding claims concerning a medication device (110) for dosing a therapeutic and/or diagnostic agent, in particular insulin.

## Revendications

1. Dispositif de médication (110) pour la distribution dosée d'un milieu fluide (112), en particulier d'un agent thérapeutique et/ou diagnostique fluide, en particulier pompe à insuline, comprenant:
a) au moins un récipient de transport et de transfert (114), dans lequel le récipient de transport et de transfert (114) présente au moins un réservoir (116) destiné à contenir le milieu fluide (112), dans lequel le réservoir (116) présente au moins un élément déplaçable (120), dans lequel l'élément déplaçable (120) est monté de façon mobile par rapport au réservoir (116) et dans lequel un positionnement relatif de l'élément déplaçable (120) par rapport au réservoir (116) détermine un espace intérieur (134) du réservoir (116) disponible pour contenir le milieu fluide (112),
b) au moins un élément de mesure (164), dans lequel l'élément de mesure (164) est conçu de façon à détecter le positionnement relatif de l'élément déplaçable (120) par rapport au réservoir (116), et
c) au moins un organe de commande (150) pour influencer une distribution du milieu fluide (112) via au moins une liaison fluide (142) vers l'espace intérieur (134),
dans lequel l'élément de mesure (164) et l'organe de commande (150) coopèrent de telle manière que la distribution du milieu fluide (112) soit influencée par le positionnement relatif détecté par l'élément de mesure (164),
**caractérisé en ce que** le dispositif de médication (110) présente au moins un boîtier (124), dans lequel le réservoir (116) est logé de façon mobile dans le boîtier (124) et dans lequel l'élément déplaçable (120) est fixé par rapport au boîtier (124), dans lequel l'élément de mesure (164) est conçu de façon à détecter un positionnement relatif du réservoir (116) dans le boîtier (124).

2. Dispositif de médication (110) selon la revendication précédente, dans lequel le dispositif de médication (110) est conçu de façon à presser l'élément déplaçable (120) contre un appui (132), de telle manière que l'élément déplaçable (120) soit poussé à l'intérieur de l'espace intérieur (134).

3. Dispositif de médication (110) selon la revendication précédente, dans lequel la liaison fluide (142) est réalisée à travers l'appui (132).

4. Dispositif de médication (110) selon l'une quelconque des revendications précédentes, dans lequel la liaison fluide (142) traverse l'élément déplaçable (120) .

5. Dispositif de médication (110) selon l'une quelconque des revendications précédentes, dans lequel l'élément de mesure (164) est conçu de façon à produire au moins un signal de mesure correspondant au positionnement relatif, dans lequel le dispositif de médication (110) présente au moins une commande (172), dans lequel la commande (172) est conçue de façon à commander et/ou réguler l'organe de commande (150) au moyen du signal de mesure.

6. Dispositif de médication (110) selon l'une quelconque des revendications précédentes, dans lequel l'élément déplaçable (120) comprend un bouchon déplaçable par rapport au réservoir (116), dans lequel l'élément de mesure (164) est conçu de façon à détecter un déplacement du bouchon dans le réservoir (116), en particulier un déplacement linéaire.

7. Dispositif de médication (110) selon l'une quelconque des revendications précédentes, dans lequel l'élément de mesure (164) est conçu de façon à détecter au moins une position d'au moins une surface limite (230) entre l'élément déplaçable (120) et le milieu fluide (112).

8. Dispositif de médication (110) selon l'une quelconque des revendications précédentes, dans lequel le dispositif de médication (110) est conçu de façon à appliquer une force à l'élément déplaçable (120) et/ou au réservoir (116), de telle manière que le milieu fluide (112) se trouve sous une pression accrue.

9. Dispositif de médication (110) selon l'une quelconque des revendications précédentes, dans lequel le dispositif de médication (110) présente au moins une unité d'énergie (126), dans lequel l'unité d'énergie (126) est conçue de façon à soumettre le milieu fluide (112) dans le réservoir (116) à une surpression, dans lequel l'unité d'énergie (126) fait partie du récipient de transport et de transfert (114).

10. Dispositif de médication (110) selon l'une quelconque des revendications précédentes, dans lequel l'organe de commande (150) présente au moins une soupape (152), dans lequel la soupape (152) est conçue de façon à influencer un écoulement du milieu fluide (112) à travers la liaison fluide (142), en particulier au choix pour l'interrompre ou le libérer.

11. Dispositif de médication (110) selon la revendication précédente, dans lequel la soupape (152) présente au moins deux positions de soupape fermées, dans lequel un écoulement du milieu fluide (112) est interrompu, lorsque la soupape (152) se trouve dans une des positions de soupape fermées.

12. Dispositif de médication (110) selon la revendication précédente, dans lequel les positions de soupape fermées comprennent des positions terminales d'un mouvement d'au moins un organe de soupape (154, 156; 182) de la soupape (152), dans lequel l'organe de soupape (154, 156; 182) peut se déplacer entre les positions de soupape.

13. Dispositif de médication (110) selon l'une quelconque des trois revendications précédentes, dans lequel la soupape (152) dans une position de repos, dans laquelle aucune force n'est exercée sur un organe de soupape (154, 156; 182) de la soupape (152), se trouve dans une position de soupape fermée, dans laquelle l'écoulement du milieu fluide (112) à travers la liaison fluide (142) est interrompu.

14. Dispositif de médication (110) selon l'une quelconque des quatre revendications précédentes, dans lequel la soupape (152) présente au moins un organe de soupape (154, 156; 182), dans lequel l'organe de soupape (154, 156; 182) peut être réglé dans deux positions de soupape statiques, dans lequel dans toutes les positions de soupape statiques un écoulement du milieu fluide (112) à travers la liaison fluide (142) est interrompu.

15. Dispositif de médication (110) selon l'une quelconque des revendications précédentes, dans lequel l'organe de commande (150) présente au moins une pompe (212) .

16. Dispositif de médication (110) selon l'une quelconque des revendications précédentes, dans lequel l'organe de soupape (150) comprend au moins deux actionneurs (158, 160; 194, 196), dans lequel la liaison fluide (142) comprend au moins une section de tuyau flexible (190), dans lequel les actionneurs (158, 160; 194, 196) sont conçus de façon à écraser la section de tuyau flexible (190) dans au moins deux régions d'écrasement différentes (178, 180; 220), dans lequel une région extensible de la section de tuyau flexible (190) est disposée entre les actionneurs (158, 160; 194, 196), dans lequel les actionneurs (158, 160; 194, 196) comprennent un actionneur côté entrée (194) et un actionneur côté sortie (196), dans lequel le dispositif de médication (110) est conçu de façon à admettre, lorsque l'actionneur côté entrée (194) est ouvert et que l'actionneur côté sortie (196) est fermé, du milieu fluide (112) sous pression dans la région extensible entre les actionneurs (194, 196), dans lequel la région extensible est étendue, dans lequel le dispositif de médication (110) est en outre conçu de façon à fermer ensuite l'actionneur côté entrée (194) et à ouvrir l'actionneur côté sortie (196), dans lequel la région extensible est détendue et une partie du milieu fluide (112) s'écoule hors de la région extensible.

17. Dispositif de médication (110) selon l'une quelconque des revendications précédentes, dans lequel le dispositif de médication (110) est de construction modulaire, dans lequel le dispositif de médication (110) comprend au moins un module de commande avec une commande (172), dans lequel le récipient de transport et de transfert (114) forme au moins un autre module du dispositif de médication (110), dans lequel le récipient de transport et de transfert (114) est réalisé de façon remplaçable.

18. Procédé de distribution dosée d'un milieu fluide (112), en particulier d'un agent thérapeutique et/ou diagnostique fluide, en particulier avec utilisation d'un dispositif de médication (110) selon l'une quelconque des revendications précédentes,
- dans lequel on utilise au moins un récipient de transport et de transfert (114), dans lequel le récipient de transport et de transfert (114) présente au moins un réservoir (116) destiné à contenir le milieu fluide (112), dans lequel le réservoir (116) présente au moins un élément déplaçable (120), dans lequel on monte l'élément déplaçable (120) de façon mobile par rapport au réservoir (116) et dans lequel un positionnement relatif de l'élément déplaçable (120) par rapport au réservoir (116) détermine un espace intérieur (134) du réservoir (116) disponible pour contenir le milieu fluide (112),
- dans lequel on détecte le positionnement relatif de l'élément déplaçable (120) par rapport au réservoir (116) au moyen d'au moins un élément de mesure (164), dans lequel on loge le réservoir (116) de façon mobile dans un boîtier (124) du dispositif de médication (110) et dans lequel on fixe l'élément déplaçable (120) par rapport au boîtier (124), dans lequel l'élément de mesure (164) détecte un positionnement relatif du réservoir (116) dans le boîtier (124),
- dans lequel on utilise en outre au moins un organe de commande (150) pour influencer une distribution du milieu fluide (112) via au moins une liaison fluide (142) vers l'espace intérieur (134),
- dans lequel l'élément de mesure (164) et l'organe de commande (150) coopèrent de telle manière que la distribution du milieu fluide (112) soit influencée par le positionnement relatif de l'élément déplaçable (120) par rapport au réservoir (116) détecté par l'élément de mesure (164).

19. Utilisation d'un dispositif de médication (110) selon l'une quelconque des revendications précédentes concernant un dispositif de médication (110) pour la distribution dosée d'un agent thérapeutique et/ou diagnostique, en particulier d'insuline.
